# EUROPEAN PATENT APPLICATION

(11) **EP 0 801 951 A2**
(43) Date of publication of application: **22.10.1997**
(21) Application number: 97108651.7
(22) Date of filing: 18.03.1993
(51) Int. Cl.: A61K 38/20, A61K 39/395

(54) **Novel uses of Il-4 and/or Il-10, and antibodies against the same**

(30) Priority: 20.08.1992 US 932900; 21.08.1992 US 933419; 21.08.1992 US 933462; 21.08.1992 US 933950
(62) Divisional of application: 93920050.7
(71) Applicant: SCHERING CORPORATION, Kenilworth New Jersey 07033 (US)
(72) Inventor: Coffman, Robert, Portola Valley, CA 94025 (US); Powrie, Fiona, 204 Mountain View, CA 94040 (US); De Vries, Jan E., Los Altos, CA 94022 (US); Rennick, Donna, Los Altos, CA 94022 (US); De Waal Malefyt, Rene, Sunnyvale, CA 94086 (US)
(74) Representative: Ritter, Stephen David

(57) **Abstract**

IL-10 alone, or optionally in combination with IL-4, are provided for use in the manufacture of pharmaceutical compositions for inhibiting delayed-type hypersensitivity reaction.

SEQUENCE LISTING

(1) GENERAL INFORMATION:

(i) APPLICANT:
(A) NAME: Schering Corporation
(B) STREET: One Giralda Farms
(C) CITY: Madison
(D) STATE: New Jersey
(E) COUNTRY: U.S.A.
(F) POSTAL CODE (ZIP): 07940-1000
(G) TELEPHONE: 201-822-7375
(H) TELEFAX: 201-822-7039
(I) TELEX: 219165

(ii) TITLE OF INVENTION: Novel Uses of IL-4 and/or IL-10,
and Antibodies Against the Same

(iii) NUMBER OF SEQUENCES: 4

(iv) COMPUTER READABLE FORM:
(A) MEDIUM TYPE: Floppy disk
(B) COMPUTER: Apple Macintosh
(C) OPERATING SYSTEM: Macintosh 6.0.5
(D) SOFTWARE: Microsoft Word 5.1a

(v) CURRENT APPLICATION DATA:
(A) APPLICATION NUMBER:
(B) FILING DATE:

(vi) PRIOR APPLICATION DATA:
(A) APPLICATION NUMBER: US 07/933950
(B) FILING DATE: 21-AUG-1992
(A) APPLICATION NUMBER: US 07/933462

(B) FILING DATE: 21-AUG-1992
(A) APPLICATION NUMBER: US 07/933419
(B) FILING DATE: 21-AUG-1992
(A) APPLICATION NUMBER: US 07/932900
(B) FILING DATE: 20-AUG-1992

(2) INFORMATION FOR SEQ ID NO:1:

(i) SEQUENCE CHARACTERISTICS:
(A) LENGTH: 160 amino acids
(B) TYPE: amino acid
(D) TOPOLOGY: linear

(ii) MOLECULE TYPE: protein

(xi) SEQUENCE DESCRIPTION: SEQ ID NO:1:

Ser Pro Gly Gln Gly Thr Gln Ser Glu Asn Ser Cys Thr His Phe Pro
1               5                   10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn Gln Asp Pro Asp Ile Lys Ala His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Lys Ser Lys Ala Val Glu Gln Val Lys Asn Ala Phe
        115                 120                 125

Asn Lys Leu Gln Glu Lys Gly Ile Tyr Lys Ala Met Ser Glu Phe Asp

    130                 135                 140

Ile Phe Ile Asn Tyr Ile Glu Ala Tyr Met Thr Met Lys Ile Arg Asn
145                 150                 155                 160

(2) INFORMATION FOR SEQ ID NO:2:
(i) SEQUENCE CHARACTERISTICS:
(A) LENGTH: 147 amino acids
(B) TYPE: amino acid
(D) TOPOLOGY: linear
(ii) MOLECULE TYPE: protein
(xi) SEQUENCE DESCRIPTION: SEQ ID NO:2:

Thr Asp Gln Cys Asp Asn Phe Pro Gln Met Leu Arg Asp Leu Arg Asp
1               5                   10                  15

Ala Phe Ser Arg Val Lys Thr Phe Phe Gln Thr Lys Asp Glu Val Asp
            20                  25                  30

Asn Leu Leu Leu Lys Glu Ser Leu Leu Glu Asp Phe Lys Gly Tyr Leu
        35                  40                  45

Gly Cys Gln Ala Leu Ser Glu Met Ile Gln Phe Tyr Leu Glu Glu Val
    50                  55                  60

Met Pro Gln Ala Glu Asn Gln Asp Pro Glu Ala Lys Asp His Val Asn
65                  70                  75                  80

Ser Leu Gly Glu Asn Leu Lys Thr Leu Arg Leu Arg Leu Arg Arg Cys
                85                  90                  95

His Arg Phe Leu Pro Cys Glu Asn Lys Ser Lys Ala Val Glu Gln Ile
            100                 105                 110

Lys Asn Ala Phe Asn Lys Leu Gln Glu Lys Gly Ile Tyr Lys Ala Met
        115                 120                 125

Ser Glu Phe Asp Ile Phe Ile Asn Tyr Ile Glu Ala Tyr Met Thr Ile
    130                 135                 140

Lys Ala Arg
145

(2) INFORMATION FOR SEQ ID NO:3:
(i) SEQUENCE CHARACTERISTICS:
(A) LENGTH: 38 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:3:
GGCTGCAGCG ACGAGCCATG GTTGCTGGGA GCGACGCG 38
(2) INFORMATION FOR SEQ ID NO:4:
(i) SEQUENCE CHARACTERISTICS:
(A) LENGTH: 35 base pairs
(B) TYPE: nucleic acid
(C) STRANDEDNESS: single
(D) TOPOLOGY: linear
(ii) MOLECULE TYPE: DNA (genomic)
(xi) SEQUENCE DESCRIPTION: SEQ ID NO:4:
TTCATCTTCT GGTACCAATC AATTGGAGTT GGTTC 35

## Description

The present invention relates to compositions and methods for treating interleukin-2 (IL-2)-dependent neoplastic cell proliferation, delayed-type hypersensitivity (DTH) reactions and inflammatory bowel disease; and for potentiating inhibition of cytokine production by T cells or increasing gamma interferon (IFN-γ) production. These compositions and methods employ interleukin-4 (IL-4), interleukin-10 (IL-10) or combinations thereof, or antagonists of IL-4 and IL-10 such as neutralizing antibodies.

### BACKGROUND OF THE INVENTION

Leukemias are a heterogeneous group of neoplasms arising from the malignant transformation of hematopoietic cells, and can be derived from either lymphoid or myeloid cell types. The transformed cells proliferate primarily in the bone marrow and lymphoid tissue where they interfere with normal hematopoiesis and immunity. They may also emigrate into the blood and infiltrate other tissues, often leading to abnormal distributions of different cell types. Examples of leukemias include, acute lymphocytic leukemia (ALL), acute myelogenous leukemia (AML), chronic myelogenous leukemia (CML), chronic lymphocytic leukemia (CLL), hairy cell leukemia, and adult T cell leukemia (ATL). Leukemias are typically identified as either acute or chronic, depending upon the nature of the clinical course.

Lymphomas, in contrast to leukemias, are neoplastic transformations of cells that reside predominantly in lymphoid tissues. Lymphomas are typically divided between Hodgkin's disease and non-Hodgkin's lymphomas. In Hodgkin's disease the majority of the cells are small lymphocytes with a T cell phenotype. More than 90% of all cases of non-Hodgkin's lymphomas are of B cell derivation.

Treatment of the majority of these diseases has not been entirely successful, and the prior art approaches have centered primarily on the use of chemotherapy and radiation. To date, however, these treatments generally fail to effect long, term remission or cure. There thus is a need for a safe, reliable method of treatment of such diseases, particularly those, the neoplastic cell proliferation of which is dependent on IL-2.

IFN-γ has been implicated as a potent activator of macrophages, which are responsible for defending a host against a variety of infectious agents and diseases. Because of the important role of IFN-γ in defense against infection, there is a need for compositions and methods that can increase the production of IFN-γ.

The immune system carries out a number of natural and adaptive immune reactions, two broad categories of which are called humoral and cellular immune responses. Humoral immunity refers broadly to antibody production and actions by B-cells including plasma cells. Cellular immunity is mediated by cells including T-cells, monocytes, macrophages and histiocytes.

T-cells may be further categorized according to various functions or markers. For instance, T-cells can be classified as T helper cells or T suppressor cells. Additionally, T-cells can be activated to become cytotoxic or to perform other more specialized functions. Normally, T-cells and B-cells have interactions that may regulate each other's activity to some extent. See, e.g., Paul (ed.) *Fundamentals of Immunology* (2d ed.) Raven Press, New York (1989).

For different antigens either cellular or humoral responses may predominate, typically in a mutually exclusive fashion. The severity of some diseases, e.g., leprosy, leishmaniasis and some types of autoimmunity may be due inappropriate dominance of one class of response over the other. Mosmann *et al., Immunol. Today 8*:223 (1987); Mosmann *et al., Ann. Rev. Immunol.* 7:145 (1989); Parish, *Transplant. Rev 13*:35 (1972); and Liew, *Immunol. Today 10*:40 (1989).

One of the mechanisms by which the immune system is regulated involves the production of proteins called cytokines. Lymphokines are cytokines produced by T-cells and some B-cells, and monokines are cytokines produced by monocytes. Cytokines, which may be glycosylated, mediate numerous immune responses.

IL-4 is a cytokine capable of stimulating production of antibody producing B-cells, which also promotes growth of killer T-cells or cytotoxic T-cells. IL-4 can also inhibit the activity of T-helper cells type 1 (Th1). This in turn may inhibit production of more B-cells or antibody production by more B-cells. Thus, IL-4 is part of an internal regulatory mechanism.

IL-10 is a cytokine capable of mediating a number of actions or effects. IL-10 has been isolated from both mouse and human cells and is involved in controlling the immune responses of different classes or subsets of T helper (Th) cells. Th cells can be divided into different subsets that are distinguished by their cytokine production profiles.

Th1 T cell clones produce interleukin-2 (IL-2) and IFN-γ, whereas Th2 cell clones secrete IL-10, IL-4 and interleukin-5 (IL-5), generally following activation by antigens or mitogenic lectins. Both classes of Th cell clones produce cytokines such as tumor necrosis factor-α (TNF-α), interleukin-3 (IL-3), and granulocyte-macrophage colony stimulating factor (GM-CSF). A third category of Th cells (Th0) produces IL-2, IFN-γ, IL-4, IL-5, TNF-α, IL-3 and GM-CSF simultaneously.

The different cytokine production patterns of Th1 and Th2 cells reflect their helper functions. Th1 cells are predominantly involved in delayed-type hypersensitivity responses, whereas Th2 cells are associated with antibody production. Since antibody (Th2 pathways) and delayed-type hypersensitivity (Th1 pathways) responses are often mutually exclusive, Th1 and Th2 cells are thought to have cross-regulatory effects. IFN-γ produced by Th1 cells inhibits proliferation of Th2 cells, and IL-10 produced by Th2 cells inhibits cytokine synthesis by Th1 cell clones, especially IFN-γ and IL-2 production.

Delayed-type hypersensitivity (DTH) is a cell-mediated immune response which is characterized by edema and cellular infiltration of the tissue, generally by T cells and monocytes and/or macrophages. Some sets of cytokines are separately associated with delayed-type hypersensitivity reactions and humoral immune responses. Cher *et al., J. Immunol. 138*:3688 (1987); and Mosmann *et al.* (1987 and 1989, *supra).* Diseases associated with these classes of response may be caused by inappropriate production of associated sets of cytokines.

Inflammatory bowel disease (IBD) refers to a group of gastrointestinal disorders characterized by a chronic non-specific inflammation of portions of the gastrointestinal tract. Ulcerative colitis and Crohn's Disease, the most prominent examples of IBD in humans, are associated with many symptoms and complications, including growth retardation in children, rectal prolapse, blood in stools (e.g., melena and/or hematochezia), wasting, iron deficiency, and anemia, e.g., iron deficiency anemia and anemia of chronic disease or of chronic inflammation.

The etiology of IBD is unclear. See, Wyngaarden and Smith (eds.) *Cecil's Textbook of Medicine* (W.B. Saunders Co. 1985), Berkow (ed.) *The Merck Manual of Diagnosis and Therapy* (Merck Sharp & Dohme Research Laboratories, 1982), and *Harrison's Principles of Internal Medicine,* 12th Ed., McGraw-Hill, Inc. (1991).

Ulcerative colitis is a chronic, non-specific, inflammatory, and ulcerative disease having manifestations primarily in the colonic mucosa. It is frequently characterized by bloody diarrhea, abdominal cramps, blood and mucus in the stools, malaise, fever, anemia, anorexia, weight loss, leukocytosis, hypoalbuminemia, and an elevated erythrocyte sedimentation rate (ESR). Complications can include hemorrhage, toxic colitis, toxic megacolon, occasional rectovaginal fistulas, and an increased risk for the development of colon cancer.

Ulcerative colitis is also associated with complications distant from the colon, such as arthritis, ankylosing spondylitis, sacroileitis, posterior uveitis, erythema nodosum, pyoderma gangrenosum, and episcleritis. Treatment varies considerably with the severity and duration of the disease. For instance, fluid therapy to prevent dehydration and electrolyte imbalance is frequently indicated in a severe attack. Additionally, special dietary measures are sometimes useful. Medications include various corticosteroids, sulphasalazine and some of its derivatives, and possibly immunosuppressive drugs.

Crohn's Disease shares many features in common with ulcerative colitis. Crohn's Disease is distinguishable in that lesions tend to be sharply demarcated from adjacent normal bowel, in contrast to the lesions of ulcerative colitis which are fairly diffuse. Additionally, Crohn's Disease predominately afflicts the ileum (ileitis) and the ileum and colon (ileocolitis). In some cases, the colon alone is diseased (granulomatous colitis) and sometimes the entire small bowel is involved (jejunoileitis). In rare cases, the stomach, duodenum, or esophagus are involved. Lesions include a sarcoid-type epithelioid granuloma in roughly half of the clinical cases.

Lesions of Crohn's Disease can be transmural, including deep ulceration, edema and fibrosis which can lead to obstruction and fistula formation as well as abscess formation. This contrasts with ulcerative colitis which usually yields much shallower lesions, although occasionally the complications of fibrosis, obstruction, fistula formation, and abscesses are seen in ulcerative colitis as well. Current treatment is similar for both diseases and includes the use of steroids, sulphasalazine and its derivatives, and immunosuppressive drugs such as cyclosporin A, mercaptopurine and azathioprine, all of which can produce strong undesired side effects.

Because of the medical importance of delayed-type hypersensitivity and inflammatory bowel disease, there is a need for improved compositions and methods for treating these conditions.

### SUMMARY OF THE INVENTION

The present invention fills the foregoing needs by providing compositions and methods for treating the above-mentioned conditions.

More particularly, this invention provides a method for inhibiting IL-2-dependent neoplastic cell proliferation comprising administering an effective amount of IL-10 to a mammal afflicted with IL-2-dependent neoplastic cell proliferation.

This invention further provides a method for increasing a patient's level of IFN-γ comprising co-administering to a patient an amount of antibodies against IL-4 and IL-10 effective to increase a patient's production of IFN-γ.

The present invention still further provides a method for treating inflammatory bowel disease in a mammal comprising administering to a mammal afflicted with inflammatory bowel disease an effective amount of IL-10.

This invention still further provides a method for potentiating inhibition of cytokine production by T cells in a mammal comprising co-administering to a mammal an effective amount of IL-4 and IL-10. The mammal thus treated is preferably a human being.

This invention still further provides a method for inhibiting a delayed-type hypersensitivity reaction in a mammal comprising administering an effective amount of IL-10. In a preferred embodiment, an effective amount of IL-4 is co-administered with the IL-10.

Preferably, human IL-4 and IL-10 are used in the foregoing methods for the treatment of human beings.

### DESCRIPTION OF THE INVENTION

All references cited herein are hereby incorporated in their entirety by reference. All nucleic acid sequences disclosed follow the normal 5' to 3' convention, as read from left to right. Standard single-letter abbreviations are used for the nucleotide bases in the sequences (37 C.F.R. § 1.822).

### Sources of Cytokines and Antibodies

As used herein, "interleukin-4" or "IL-4" means a protein which (a) has an amino acid sequence substantially identical to the sequence of mature human IL-4 disclosed in Fig. 1C of International Patent Application Publication No. WO 87/02990 and (b) has biological activity that is common to native IL-4. Substantial identity of amino acid sequences means that the sequence of another IL-4 compared to the sequence disclosed by the published patent application is identical or differs by one or more amino acid alterations (deletions, additions, substitutions) that do not substantially impair biological activity.

IL-4 is commercially available from various sources, such as Genzyme Corporation, Cambridge, MA., or it can be prepared by known methods using natural sources or recombinant DNA methodologies [Sheehan *et al., J. Immunol. 142*:884 (1989) and Starnes *et al., J. Immunol., 145*:4185 (1990)].

Alternatively, oligonucleotide probe mixtures based on known IL-4 nucleotide sequences can be used to identify DNA encoding IL-4 in genomic or cDNA libraries prepared by standard methods. DNA thus identified can be excised from the library by restriction endonuclease cleavage or prepared using appropriate primers and the polymerase chain reaction (PCR) method [Saiki *et al., Science 239*:487 (1988)], sequenced and expressed in a eukaryotic expression system or (following intron deletion by standard methods if necessary) in a prokaryotic or eukaryotic expression system. Of course, both cDNA and genomic DNA libraries can be screened by the application of standard expression cloning methods, instead of by the use of oligonucleotide probes or PCR. IL-4 thus produced is detectable through the use of known methods such as immunochemical or bioassay methods.

The IL-4 used will preferably be human recombinant IL-4. Glycosylated IL-4 be used (e.g., recombinant IL-4 produced in a eukaryotic expression system) as well as the unglycosylated protein (e.g., chemically synthesized or produced in a bacterial expression system.

As used herein, "interleukin-10" or "IL-10" is defined as a protein which (a) has an amino acid sequence of mature (e.g., lacking a secretory leader sequence) IL-10 substantially as disclosed in International Application No. WO 91/00349, and (b) has biological activity that is common to native IL-10. Both glycosylated (e.g. produced in eukaryotic cells such as CHO cells) and unglycosylated (e.g., chemically synthesized by standard methods or produced in *E. coli)* IL-10 can be used. Also included are muteins and other analogs, including BCRF1 (Epstein Barr Virus viral IL-10; also called simply viral IL-10) protein, which possess the biological activity of IL-10. International Application Publication No. WO 91/00349 describes a number of *in vitro* assays suitable for measuring IL-10 activity. A number of preferred modifications of IL-10 are described in this application.

IL-10 suitable for use in the invention can be obtained from a number of sources. For example, it can be isolated from culture media of activated T-cells capable of secreting the protein. Additionally, the IL-10 or active fragments thereof can be chemically synthesized using standard techniques as known in the art. See, e.g., Merrifield, Science *233*:341-47 (1986) and Atherton *et al., Solid Phase Peptide Synthesis, A Practical Approach,* 1989, I.R.L. Press, Oxford.

Recombinant human IL-10 is also an article of commerce, available for purchase, e.g., from PeproTech, Inc., Rocky Hill, NJ.

Alternatively, recombinant IL-10 can be produced as described above for IL-4, using known human IL-10 or viral IL-10 sequence information. Methods for making recombinant human IL-10 are disclosed, e.g., in International Application Publication No. WO 91/00349. Clones comprising sequences encoding human IL-10 were deposited with the American Type Culture Collection (ATCC), Rockville, Maryland, on December 20, 1989 and assigned Accession Numbers 68191 and 68192. The cloning and expression of a viral IL-10 (BCRFI protein) from Epstein Bar virus has been disclosed by Moore *et al. [Science 248*:1230 (1990)].

Preferably, human IL-10 is used for the treatment of humans, although viral IL-10 or IL-10 from some other mammalian species can be used instead. Most preferably, the IL-10 used is recombinant human IL-10.

IL-4 and IL-10 from whatever source can be purified using standard methods including but not limited to acid or salt precipitation, ion-exchange chromatography, metal chelate chromatography, gel filtration, fast protein liquid chromatography (FPLC), high performance liquid chromatography (HPLC), preparative disc gel or curtain electrophoresis, isoelectric focusing, low temperature organic solvent fractionation, countercurrent distribution and immunoaffinity chromatography.

Antibodies can be prepared against IL-4 and IL-10 using standard methods. As used herein, the word "antibody" refers to both polyclonal and monoclonal antibodies (McAbs). It also includes whole immunoglobulins and antigen binding fragments thereof.

Polyclonal antibodies can be produced by immunizing a host animal such as a rabbit, rat, goat, sheep, mouse, etc. with IL-4 or IL-10. Preferably, one or more booster injections are given after the initial injection, to increase the antibody titer. Blood is then drawn from the animal and serum is prepared and screened by standard methods such as enzyme-linked immunosorbent assay (ELISA) using the polypeptide as the antigen. Immunoglobulin fractions can be prepared by standard methods.

Preferably, the immunogenicity of the IL-4 or IL-10 is increased by combination with one of the many known adjuvants and/or by conversion to a larger form prior to immunization, i.e., by cross-linking using standard methods.

Serum produced from animals thus immunized can be used directly. Alternatively, the IgG fraction can be separated from the serum using standard methods such as plasmaphoresis or adsorption chromatography using IgG specific adsorbents such as immobilized Protein A.

Monoclonal antibodies, which are preferred for use in this invention, can be prepared using standard methods, e.g., as described by Kohler *et al. [Nature 256*:495 (1975); *Eur. J. Immunol. 6*:511 (1976)]. Essentially, an animal is immunized as described above to produce antibody-secreting somatic cells. These cells are then removed from the immunized animal for fusion to myeloma cells.

Somatic cells with the potential to produce antibodies, particularly B cells, are suitable for fusion with a myeloma cell line. These somatic cells may be derived from the lymph nodes, spleens and peripheral blood of primed animals. Rat spleen cells are often used, in part because these cells produce a relatively high percentage of stable fusions with mouse myeloma lines. It would be possible, however, to use human, mouse, rabbit, sheep, goat or other cells instead.

Specialized myeloma cell lines have been developed from lymphocytic tumors for use in hyridoma-producing fusion procedures [Kohler and Milstein, *Eur. J. Immunol. 6*:511. (1976); Shulman *et al., Nature 276*:269 (1978); Volk *et al., J. Virol. 42*:220 (1982)]. Methods for generating hybrids of antibody-producing spleen or lymph node cells and myeloma cells usually involve mixing somatic cells with myeloma cells in a 10:1 proportion (although the proportion may vary from about 20:1 to about 1:1), respectively, in the presence of an agent or agents (chemical, viral or electrical) that promotes the fusion of cell membranes. Fusion methods have been described by Kohler and Milstein, *supra,* Gefter *et al. [Somatic Cell Genet. 3*:231 (1977)], and Volk *et al. (J. Virol. 42*:220 (1982)]. The fusion-promoting agents used by those investigators were Sendai virus and polyethylene glycol (PEG).

Because fusion procedures produce viable hybrids at very low frequency (e.g., when spleens are used as a source of somatic cells, only one hybrid is obtained for roughly every 1 x 10⁵ spleen cells), it is essential to have a means of selecting the fused cell hybrids from the remaining unfused cells, particularly the unfused myeloma cells. A means of detecting the desired antibody-producing hybridomas among other resulting fused cell hybrids is also necessary.

Generally, the selection of fused cell hybrids is accomplished by culturing the cells in media that support the growth of hybridomas but prevent the growth of the unfused myeloma cells, which normally would go on dividing indefinitely. The somatic cells used in the fusion do not maintain long-term viability in *in vitro* culture and hence do not pose a problem. For example, myeloma cells lacking hypoxanthine phosphoribosyl transferase (HPRT-negative) can be used. Selection against these cells is made in hypoxanthine/aminopterin/thymidine (HAT) medium, a medium in which the fused cell hybrids survive due to the HPRT-positive genotype of the spleen cells. The use of myeloma cells with different genetic deficiencies (drug sensitivities, etc.) that can be selected against in media supporting the growth of genotypically competent hybrids is also possible.

Several weeks are required to selectively culture the fused cell hybrids. Early in this time period, it is necessary to identify those hybrids which produce the desired antibody, so that they may subsequently be cloned and propagated. Generally, around 10% of the hybrids obtained produce the desired antibody, although a range of from about 1 to about 30% is not uncommon.

The detection of antibody-producing hybrids can be achieved by any one of several standard assay methods, including enzyme-linked immunoassay and radioimmunoassay techniques which have been described in the literature [see, e.g., Kennet *et al.* (editors), *Monoclonal Antibodies and Hybridomas: A New Dimension in Biological Analyses,* pp. 376-384, Plenum Press, New York (1980)].

Once the desired fused cell hybrids have been selected and cloned into individual antibody-producing cell lines, each cell line may be propagated in either of two standard ways. A suspension of the hybridoma cells can be injected into a histocompatible animal. The injected animal will then develop tumors that secrete the specific monoclonal antibody produced by the fused cell hybrid. The body fluids of the animal, such as serum or ascites fluid, can be tapped to provide monoclonal antibodies in high concentration. Alternatively, the individual cell lines may be propagated *in vitro* in laboratory culture vessels. The culture medium containing high concentrations of a single specific monoclonal antibody can be harvested by decantation, filtration or centrifugation, and subsequently purified.

Once a hybridoma producing the desired monoclonal antibody is obtained, techniques can be used to produce interspecific monoclonal antibodies wherein the binding region of one species is combined with a non-binding region of the antibody of another species [Liu *et al., Proc. Natl. Acad. Sci.* USA *84*:3439 (1987)]. For example, the CDRs from a rodent monoclonal antibody can be grafted onto a human antibody framework, thereby "humanizing" the rodent antibody [Riechmann *et al., Nature 332*:323 (1988)]. More particularly, the CDRs can be grafted into a human antibody variable region with or without human constant regions. Such methodology has been used, e.g., to humanize a mouse monoclonal antibody against the p55 (Tac) subunit of the human interleukin-2 receptor [Queen *et al., Proc. Natl. Acad. Sci.* USA *86*:10029 (1989)].

Alternatively, human monoclonal antibodies can be prepared using IL-4 or IL-10 and methods described by Banchereau *et al. [Science 251*:70 (1991)].

The present invention also encompasses antibody binding fragments such as Fab, F(ab')₂, Fv fragments, etc. The use and generation of fragments of antibodies is well known, e.g., Fab fragments [Tijssen, *Practice and Theory of Enzyme Immunoassays* (Elsevier, Amsterdam, 1985)], Fv fragments [Hochman *et al., Biochemistry 12*:1130 (1973); Sharon *et al., Biochemistry 15*:1591 (1976); Ehrlich *et al.,* U.S. Patent No. 4,355,023] and antibody half molecules (Auditore-Hargreaves, U.S. Patent No. 4,470,925).

Preferably, the antibodies and antibody fragments used in this invention will specifically bind to and neutralize the biological activity of the IL-4 or IL-10 against which they were prepared.

### Inhibition of IL-2-dependent Neoplastic Cell Proliferation

As is shown in an Example below, human and viral IL-10 have direct effects on human T cells. The proliferative responses of human Th0-, Th1-, and Th2-like cells were all found to be inhibited by IL-10 when L cells transfected with FcγRII (CD32) were used in combination with either anti-CD3 monoclonal antibodies, or a mitogenic pair of anti-CD2 monoclonal antibodies. In addition, antigen-specific proliferative responses of human T-cell clones were inhibited by IL-10 when mouse L cells transfected with the relevant class II MHC molecules were used as APCs. IL-10 did not affect class II MHC expression in this system, since the constitutive expression of the transfected class II MHC genes is under the control of the SV40 promoter.

The inhibitory effects of IL-10 are due primarily to a direct effect of IL-10 on the T cell clones via inhibition of IL-2 production at the mRNA level. The inhibitory effects were specific for IL-2, since IL-10 did not affect the production of IFN-γ, IL-4, IL-5, and GM-CSF. The interaction of IL-10 with an IL-10 receptor apparently triggers a signalling pathway that specifically inhibits IL-2 synthesis.

Other assays can also be used to demonstrate the efficacy of the compositions of the present invention. For instance, nude mouse xenograft models can be used. Such models have been used to test a wide variety of chemotherapeutic agents [see, Howard *et al., Cancer Res. 51*:3274 (1991) and McLemore *et al., Cancer Res. 47*:5132 (1987)].

Such cell proliferation may be associated with a leukemia such as B cell CLL. Mammals treated by the method of this invention are preferably human patients afflicted with IL-2-dependent neoplastic cell proliferation.

The method of this invention for inhibiting IL-2-dependent neoplastic cell proliferation comprises administering to a mammal, preferably a human patient suffering from neoplastic cell proliferation, a therapeutically effective dose of IL-10. A therapeutically effective dose of a second biologically active agent may also be administered.

Pharmaceutical compositions comprising IL-10 and a physiologically acceptable carrier are typically administered intravenously. The IL-10 may also be encapsulated in a liposome.

As used herein, the term "IL-2 dependent neoplastic cell proliferation" is defined as any new or abnormal growth of tissue in which the growth is uncontrolled, progressive, and depends upon IL-2 for continued proliferation. The growth can be either benign or malignant. Malignant growth is typically characterized by greater degree of anaplasia (dedifferentiation) in the neoplastic cells. The IL-2-dependent neoplastic cells are typically derived from lymphoid or myeloid lines.

IL-2 is produced by T cells and has multiple activities on various cell types, including T cells, NK cells and B cells. IL-2 has been shown to act as a T cell growth factor and to enhance the production of cytokines by T cells when stimulated in the presence of IL-2. IL-2 activates natural killer (NK) cells to kill target cells in a MHC non-specific manner. In addition, IL-2 is involved in the expansion of committed cells, thereby enhancing antibody production by these cells.

Based on these activities of IL-2, the inhibition of IL-2 production by IL-10 could be important in several disease situations. For example, it could serve as a means to reduce IL-2 driven proliferation of T cells in situations where T cell proliferation and growth is undesirable, such as in autoimmune diseases like autoimmune thyroiditis, insulin-dependent diabetes, rheumatoid arthritis, systemic lupus erythmatosis and multiple sclerosis.

In addition, the capacity of IL-10 to reduce IL-2-driven T cell expansion has potential clinical utility in the treatment of acute graft versus host disease and chronic inflammatory diseases such as inflammatory bowel disease, sarcoid and pulmonary fibrosis. Moreover, by inhibiting the IL-2-driven proliferation of allergen-specific T cells, which are strong producers of IL-4 and IL-5, IL-10 can be used to treat allergic diseases such as asthma and atopic dermatitis.

Diseases involving neoplastic growth that can be treated with the pharmaceutical compositions of the present invention include leukemias known to be dependent on IL-2 such as B cell CLL and ATL. Pharmaceutical compositions of the invention are suitable for use in a variety of drug delivery systems. For a brief review of present methods for drug delivery, see Langer, *Science 249*:1527 (1990). Methods for preparing administrable compounds will be known or apparent to those skilled in the art and are described in more detail in for example, *Remington's Pharmaceutical Science,* 17th ed., Mack Publishing Company, Easton, PA (1985).

The IL-10 used in this invention may be prepared as formulations in pharmaceutically acceptable media, for example, saline, phosphate buffered saline (PBS), Ringer's solution, dextrose/saline, Hank's solution, and glucose. The compositions may contain pharmaceutically acceptable auxiliary substances as required to approximate physiological conditions, such as buffering agents, tonicity adjusting agents, wetting agents, detergents, and the like. Additives may also include additional active ingredients, e.g., bactericidal agents, or stabilizers. The amount administered to the patient will vary depending upon what is being administered, the purpose of the administration, such as prophylaxis or therapy, the state of the host, the manner of administration, and the like.

The pharmaceutical compositions are typically intended for transdermal or parenteral administration, *e.g.,* intravenously, subcutaneously, or intramuscularly. Orally administrative forms are also desired and can be provided by modifying the composition to bypass the stomach environment. The composition can be used for prophylactic and/or therapeutic treatment. Preferably, the pharmaceutical compositions are administered intravenously. Thus, the invention provides compositions which comprise an IL-10 polypeptide dissolved or suspended in an acceptable carrier, preferably an aqueous carrier. These compositions may be sterilized by conventional sterilization techniques, or may be sterile filtered.

The resulting aqueous solutions may be packaged for use as is, or lyophilized, the lyophilized preparation being combined with a sterile aqueous carrier prior to administration. The IL-10 may also be administered with a second biologically active agent, such as a standard chemotherapeutic agent. Such agents include but are not limited to vincristine, daunorubicin, L-asparaginase, mitoxantrone and amsacrine.

In therapeutic applications, the pharmaceutical compositions are administered to a patient in an amount sufficient to inhibit IL-2-dependent neoplastic cell proliferation. An amount adequate to produce the desired effect is defined as a "therapeutically effective dose.' The therapeutically effective dose of IL-10 will vary according to, for example, the particular use for which the treatment is made, the manner of administration, the health and condition of the patient, and the judgment of the prescribing physician. For example, the dose for continuous infusion will typically be the range of about 500 ng to about 800 µg per day for a 70 kg patient, preferably between about 10 µg and about 300 µg. The dose will typically be between 700 ng/kg/day and 16 µg/kg/day.

The concentration of IL-10 in the pharmaceutical formulations can vary widely, *i.e.,* from about 10 µg to about 5 mg/ml, preferably between about 100 µg and about 2 mg/ml. The concentration will usually be selected primarily by fluid volumes, viscosities, etc., in accordance with the particular mode of administration selected. Thus, a typical pharmaceutical composition for intravenous infusion could be made up to contain 250 ml of dextrose/saline solution and 2.5 mg IL-10.

For solid compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For oral administration, a pharmaceutically acceptable nontoxic composition is formed by incorporating normally employed excipients, such as those carriers previously listed, and generally 10-95% of active ingredient, that is, an IL-10 polypeptide of the invention, preferably 25%-75%.

For aerosol administration, the IL-10 is preferably supplied in finely divided form along with a surfactant and propellant. Typical percentages of IL-10 are 0.01%-20% by weight, preferably 1%-10%. The surfactant must, of course, be nontoxic, and preferably soluble in the propellant. Representative of such agents are the esters or partial esters of fatty acids containing from 6 to 22 carbon atoms such as caproic, octanoic, lauric, palmitic, stearic, linoleic, linolenic, olesteric and oleic acids with an aliphatic polyhydric alcohol or its cyclic anhydride such as, for example, ethylene glycol, glycerol, erythritol, arbitol, mannitol, sorbitol, the hexitol anhydrides derived from sorbitol, and the polyoxyethylene and polyoxypropylene derivatives of these esters. Mixed esters, such as mixed or natural glycerides may be employed.

The surfactant may constitute 0.1%-20% by weight of the composition, preferably 0.25-5%. The balance of the composition is ordinarily propellant. Liquified propellants are typically gases at ambient conditions, and are condensed under pressure. Among suitable liquified propellants are the lower alkanes containing up to 5 carbons, such as butane and propane; and preferably fluorinated or fluorochlorinated alkanes. Mixtures of the above may also be employed. In producing the aerosol, a container equipped with a suitable valve is filled with the appropriate propellant, containing the finely divided peptide(s) and surfactant. The ingredients are thus maintained at an elevated pressure until released by action of the valve.

To enhance the serum half-life, the IL-10 may be encapsulated, introduced into the lumen of liposomes, prepared as a colloid, or other conventional techniques may be employed which provide an extended lifetime of the peptides. Thus, in certain embodiments, the IL-10 may be encapsulated in a liposome. A variety of methods are available for preparing liposomes, as described in, e.g., Szoka *et al., Ann. Rev. Biophys. Bioeng. 9*:467 (1980), U.S. Pat. Nos. 4, 235,871, 4,501,728, and 4,837,028.

Following preparation, the liposomes may be sized to achieve a desired size range and relatively narrow distribution of liposome sizes. Several techniques are available for sizing liposomes to a desired size, one of which is described in U.S. Patent No. 4,737,323.

Even under the most efficient encapsulation methods, the initial sized liposome suspension may contain up to 50% or more drug in a free (non-encapsulated) form. Several methods are available for removing non-entrapped compound from a liposome suspension. In one method, the liposomes in the suspension are pelleted by high-speed centrifugation leaving free compound and very small liposomes in the supernatant. Another method involves concentrating the suspension by ultrafiltration, then resuspending the concentrated liposomes in a replacement medium. Alternatively, gel filtration can be used to separate large liposome particles from solute molecules.

Following the above treatment, the liposome suspension is brought to a desired concentration for use in intravenous administration. This may involve resuspending the liposomes in a suitable volume of injection medium, where the liposomes have been concentrated, for example by centrifugation or ultrafiltration, or concentrating the suspension, where the drug removal step has increased total suspension volume. The suspension is then sterilized by filtration as described above. The liposomes comprising the peptides of the invention may be administered parenterally as described above.

### Increased Production of IFN-γ

This method of the present invention is particularly useful where a patient suffers from an infectious disease wherein the patient's immune system inhibits the production of IFN-γ due to production of IL-4 and IL-10. Representative diseases include visceral leishmaniasis; lepromatous leprosy; and fungal infections. Representative fungal infections include genera of fungi selected from the group consisting of: *Candida; Paracoccidioidomycosis; Blastomyces;* and *Cryptospiroidium.*

A number of infectious organisms benefit either directly or indirectly from low levels of IFN-γ. These infections are often chronic and are typified by an imbalance in Th2 versus Th1 responses to the infectious agent. The result of this imbalance is an inhibition of IFN-γ production by the Th1 cells due to IL-4 and IL-10 production by the Th2 cells.

Antibodies used in this invention are preferably autologous for the patient. Non-self antibodies derived from cells of the same species are also useful. Antibodies of different species can be used, but means to control possible adverse immune reactions (HAMA) may be employed. For example, humanized rat antibodies can minimize immune responses in human patients, as can various binding fragments. The antibodies will preferably have binding constants which approximate or exceed the affinity of IL-4 and IL-10 for their natural receptors.

Antibodies having a binding constant greater than 100-fold less than these cytokines for their corresponding receptors are preferred. Binding comparisons are carried out using standard equilibrium methods. The basic technology is described in Chpt 25 of Vol. 1: *Immunochemistry,* Ed. D.M. Weir, 4th Ed. 1986, Blackwell Scientific Publ. 25.1-25.30. Alternatively, one can use an assay for determining the molar excess of antibody which neutralizes a defined amount of IL-4 or IL-10 in a standard in *vitro* bioassay. Examples of such assays are found in Mosmann *et al., J. Immunol. Methods 116*:151 (1989) (IL-4) and Fiorentino *et al., J. Exp. Med. 170*:2081 (1989). A reasonable range are those antibodies which neutralize a given amount of IL-4 or IL-10 in a 10 to 1,000 fold excess.

As used herein, the term "co-administered" means that the antibodies against IL-4 and IL-10 are administered close enough together in time so that they are present in the patient together. The particular order of administration is not critical, and the antibodies can be administered simultaneously or separately, either by premixing or by separate administration.

The means of administration is typically parenteral, preferably intravenous. The antibodies can be infused into the patient using standard intravenous techniques. The antibodies are first suspended into a sterile, physiologically-compatible media, such as phosphate buffered saline. Pharmaceutically acceptable excipients such as lecithin, glucose, dextrose, antibiotics may also be included with the antibodies.

The amount of antibody co-administered ranges from 1-10 mg/kg body weight per antibody. It is desired that the antibodies be administered in an amount which provides circulating levels of about 1 to 150 µg/ml, preferably 10 to 100 µg/ml of sera, for each antibody. The antibodies typically have a 2-7 day half-life, and repeated administration is appropriate when levels of either antibody fall below the desired levels. Antibody levels in serum samples can be measured using conventional immunoassays, preferably ELISA assays.

Although monoclonal antibodies against IL-4 and IL-10 are used to illustrate this embodiment of the invention, other antagonists can be used as well. For example, antibodies that specifically bind to IL-4 or IL-10 receptors and thereby inhibit cytokine binding can be used. Similarly, soluble forms of the IL-4 or IL-10 receptor lacking the transmembrane domains can be used. Finally, mutant antagonist forms of IL-4 and IL-10 can be used which bind strongly to the corresponding receptors but essentially lack biological activity. One such IL-4 mutant antagonist, in which the tyrosine residue at position 124 of wild-type human IL-4 has been replaced by an aspartic acid residue, has been described by Kruse *et al. [EMBO J. 11*:3237 (1992)].

The antagonists are preferably co-administered intravenously, using a sterile aqueous mixture. The mixture can comprise any pharmaceutically acceptable excipient, such as sterile buffers, saline, antibiotics and the like.

Treatment is typically terminated when the infection is under control as measured by the patients' clinical response. Conventional antibiotic therapies may also be used in conjunction with this invention.

### Inflammatory Bowel Disease

The term "inflammatory bowel disease," or "IBD," is used herein to include ulcerative colitis and Crohn's Disease. The administration of IL-10 to treat IBD is preferably parenteral, such as intravascular. Most preferably, the administration is intravenous and the mammal treated is a human being.

The amount of IL-10 administered will generally range from about 1 µg to about 100 mg per kilogram of body weight. Often, the range will be from about 10 µg to about 1000 µg per kilogram of body weight. Most preferably, a dose of from about 50 µg to about 100 µg per kilogram of body weight will be administered.

The IL-10 can be administered alone or co-administered in combination with one or more additional therapeutic agents. Examples of such agents useful in controlling episodic inflammation of intestinal tissue include corticosteroids, sulphasalazine, derivatives of sulphasalazine, immunosuppresive drugs such as cyclosporin A, mercaptopurine, and azathioprine, and other cytokines. The co-administration can be sequential or simultaneous. Co-administration generally means that the multiple (two or more) therapeutics are present in the recipient during a specified time interval. Typically, if a second agent is administered within a half-life of a first agent, the two agents are considered co-administered.

This invention further provides a method for predicting a mammal's predisposition for development of an inflammatory condition characterized by suboptimal levels of IL-10 comprising assaying a sample taken from the mammal for an IL-10 level. Suboptimal levels include undetectable amounts. A detectable level could be compared to a known normal level of IL-10. Alternatively, one can assay for inflammatory mediators such as IL-1, IL-6, TNF, and IFN-γ using commercially available diagnostic kits.

Overproduction of one of these mediators can indicate that insufficient amounts of IL-10 are available. Preferably, blood is the sample source. The method allows for prediction of predisposition to a number of inflammatory conditions, such as an IBD or an anemia, an arthritis, a dermatitis, or other syndrome associated with an IBD.

Pharmaceutical compositions are also provided. A composition for administration to a mammal having an IBD, such as ulcerative colitis or Crohn's Disease, includes an amount of IL-10 effective to ameliorate at least one symptom or sign of the IBD in the mammal, and a pharmaceutically acceptable carrier as described above.

Generally, the term "symptom" refers any subjective evidence of disease or of a patient's condition. This includes evidence as perceived by the patient. Examples of symptoms of IBD include diarrhea, abdominal pain, fever, melena, hematochezia, and weight loss. The term "sign" refers generally to any objective evidence of a disease or condition, usually as percieved by an examining physician or features which would reveal themselves on a laboratory evaluation or other tests such as an ultrasonic study or a radiographic test.

Some examples of signs of IBD include abdominal mass, glossitis, aphthous ulcer, anal fissure, perianal fistula, anemia, malabsorption, and iron deficiency. Occasionally, signs and symptoms overlap. For example, the patient complains of bloody stools (a symptom), and a laboratory test of a stool sample is positive for blood (a sign).

Pharmaceutical compositions of this embodiment of the invention are preferably in a form suitable for parenteral administration. Preferably, the effective amount is a unit dose presented in an ampoule. Alternatively, the effective amount could be presented in a vial containing multiple doses or it could be offered in some other form. Examples of pharmaceutically acceptable additives include vehicles such as aqueous vehicles, buffers, diluents, antimicrobials, and preservatives.

Biological assays of the cytokines themselves can also be used to determine IL-10 activity. A biological assay for human lymphotoxin is disclosed in Aggarwal, *Methods in Enzymology 116*:441 (1985) and Matthews, *et al.,* (Clemens, *et al.,* eds.), *Cytokines and Interferons; A Practical Approach:* pp. 221-225 (IRL Press, Washington, D.C. 1987). Human IL-2 and GM-CSF can be assayed with factor-dependent cell lines CTLL-2 and KG-1, available from the ATCC under accession numbers TIB 214 and CCL 246, respectively. Human IL-3 can be assayed by its ability to stimulate the formation of a wide range of hematopoietic cell colonies in soft agar cultures, e.g., as described by Metclaf, "The Hemopoietic Colony Stimulating Factors" (Elsevier, Amsterdam, 1984). IFN-γ can be quantified with anti-viral assays, e.g., Meager in Clemens, *et al.* (eds.) at pp. 129-147. Monitoring of these cytokines can provide useful information on when an effective amount of IL-10 has been administered. Immunochemical assays such as ELISA can also be used.

The IL-10 may be administered in aqueous vehicles such as water, saline or buffered vehicles with or without various additives and/or diluting agents. Alternatively, a suspension, such as a zinc suspension, can be prepared to include the IL-10. Such a suspension can be useful for subcutaneous (SQ) or intramuscular (IM) injection. The proportion of IL-10 and additive can be varied over a broad range so long as both are present in effective amounts. On a per-dose basis, the amount of the IL-10 could range from about 1 microgram (µg) to about 100 milligrams (mg).

The total daily dose typically ranges from about 1 µg to about 100 mg per kilogram of body weight. Preferably, the dose is selected from a range of about 10 µg to about 1000 µg per kilogram of body weight. More preferably, the dose is selected from a range of about 50 µg to about 100 µg per kilogram of body weight. Dosages are administered on a schedule which produces the desired therapeutic effect and can be periodic over a short or longer term, or irregular as dictated by the episodic nature of such inflammations.

Compositions may be ingested orally or injected into the body. Formulations for oral use include compounds to protect the polypeptides from proteases which occur in the gastrointestinal tract. Injections are usually intramuscular, subcutaneous, intradermal or intravenous. Alternatively, intra-articular injection or other routes could be used in appropriate circumstances. Additionally, compositions including the IL-10 may be implanted into a patient or injected using a drug delivery system. See, for example, Urquhart, *et al., Ann. Rev. Pharmacol. Toxicol.* 24:199 (1984); Lewis, ed. "Controlled Release of Pesticides and Pharmaceuticals" (Plenum Press, New York, 1981); U.S.. Patents Nos. 3,773,919 and 3,270,960.

Preferably, the peptide is administered parenterally, and preferably in a unit dosage injectable form. Examples of an injectable form include solutions, suspensions and emulsions. More preferably, an effective amount of IL-10 is administered intravenously.

The phrase "effective amount" is defined herein to mean an amount sufficient to ameliorate a symptom or sign of an autoimmune condition or of an undesirable or inappropriate inflammatory or immune response. Typical mammalian hosts will include mice, rats, cats, dogs, and primates, including human beings. An effective amount for a particular patient may vary depending on factors such as the condition being treated; the overall health of the patient; the method, route and dose of administration, and the severity of side affects.

Determination of the appropriate dose is made by the clinician using parameters known in the art. Generally, the dose begins with an amount somewhat less than the optimum dose and it is increased by small increments thereafter until the desired or optimum effect is achieved. *See* generally *The Merck Manual* § 269 "Pharmacokinetics and Drug Administration." TNFα, IFN-γ, IL-1, and IL-6 levels would be important indicators of when an effective dose is reached. Preferably, IL-10 derived from the mammalian species treated will be used.

Typically, the IL-10 is injected in association with a pharmaceutical carrier such as normal saline, Ringer's solution, dextrose solution, and other aqueous carriers known in the art. Appropriate non-aqueous carriers may also be used and examples include fixed oils and ethyl oleate. A preferred carrier is 5% dextrose in saline. Frequently, it is desirable to include additives in the carrier such as buffers and preservatives or other substances to enhance isotonicity and chemical stability.

The total daily dose may be given as a single injection or a continuous infusion. Alternatively, it may be divided into several smaller doses for bolus intravenous administration or administration by some other route such as intramuscular injection. Preferably, the IL-10 is administered as an intravenous bolus.

The IL-10 may be administered alone or in combination with other therapeutic agents. Examples of such agents in an inflammatory bowel indication include corticosteroids, sulphasalazine, derivatives of sulphasalazine, and selected cytotoxic drugs such as cyclosporin A, mercaptopurine, and azathioprine. Typically, the multiple medications are separately infused or injected sequentially. In appropriate circumstances, multiple medications are mixed and infused or injected simultaneously together, e.g., IL-10 with other cytokines, steroids, or other therapeutic reagents.

It should be noted that IBD symptoms may be episodic, so effective treatment may be achieved by administration of small doses at opportune moments. Alternatively, small amounts of continuous administration may provide long periods of episode-free health for suffering target animals.

The co-administration can be simultaneous or sequential. Generally, "co-administering" means that the cytokines are both present in the recipient during a specified time interval. Typically, if a second cytokine is administered within a half life of the first cytokine to be administered, the two cytokines were co-administered. Preferably, the co-administration is parenteral, and more preferably it is intravenous. The effective amount is selected from a range from about 15 µg to about 1500 µg per kilogram of body weight of the mammal.

### Inhibition of Delayed-type Hypersensitivity Reactions

This embodiment of the invention provides an *in vivo* method of inhibiting a reaction of delayed-type hypersensitivity in a mammal comprising administering to the mammal an effective amount of IL-10. An effective amount of IL-4 can be co-administered with the IL-10 to the mammal, and the mammal is typically a human being. The co-administration of the IL-4 and IL-10 can be simultaneous or sequential. Usually, the administration is parenteral, preferably intravascular.

Additionally, a method of inhibition of cellular migration to a site of inflammation in the body of the mammal is provided by this invention. The cellular migration refers to recruitment or attraction of the mammal's cellular immune response including lymphocytes, monocytes, and macrophages to a site of inflammation. Also, a method of inhibition of swelling in tissues of the mammal is provided.

The above methods can include administration of IL-4 and/or IL-10 which is parenteral, such as intravascular, or which is in the proximity of local inflammation. An effective amount of either IL-4 or IL-10 is usually selected from a range from about 15 µg to about 1500 µg per kilogram of body weight of the mammal.

The present invention further provides an *in vivo* method of limiting a reaction of delayed-type hypersensitivity in a mammal comprising co-administering to the mammal an effective amount of each of IL-4 and IL-10, wherein the co-administration is parenteral and the effective amount is selected from a range from about 15 µg to about 1500 µg per kilogram of body weight of the mammal. The effective amount can be selected from a range from about 100 µg to 1000 µg.

Additionally, this invention includes a kit for potentiating IL-10 mediated inhibition of cytokine production by T cells in a mammal. The kit comprises an ampoule containing a unit dose of a mixture of IL-4 and IL-10. .Instead of an ampoule, some other container could be used. Examples include a vial, a plastic bag, a glass test tube, and the like. Preferably, the mixture of IL-4 and IL-10 is in a form suitable for parenteral administration. The unit dose is typically selected from a range from about 15 µg to about 1500 µg of each of IL-4 and IL-10 per kilogram of body weight of the mammal.

An underlying concept of the invention is the cooperative effect of IL-4 and IL-10 on T-cell activity. The invention represents the first time that this cooperation has been identified relating to T-cells specifically. Previous data has indicated effects on macrophages or monocytes and the effector role of macrophages. The additive effect of IL-4 and IL-10 on T-cell activity is surprising and supports additional therapeutic uses particularly with regard to cell mediated immunity. Because the combination of IL-4 and IL-10 potentiates the IL-10 mediated inhibition of T-cell cytokine production, the invention should be useful whenever it is desirable to control T-cell response relating to cytokine production. Examples include effects on delayed type hypersensitivity, cell mediated inflammation and T-cell proliferation and activation.

For instance, the autoimmune disease diabetes mellitus type I is characterized by islet cell infiltration with lymphocytes, macrophages and plasma cells. Suppression of cytokine production by T-cells may ameliorate the symptoms and islet cell destruction of diabetes. Additionally, the invention may play a role in treatment of cell-mediated immune responses relating to organ transplantation and grafts.

Kidney rejection is known to include cell mediated immunity. Cytokines in the region of the transplanted kidney cause cell activation, proliferation, aggregation, fibrogenesis, inhibition of growth and cytotoxicity. Similarly, the invention may play a beneficial role in the treatment of GVHD. Furthermore, sarcoidosis, a lymphoproliferative disorder is associated with evidence of deceased DTH and activated T or helper cells with subsequent granuloma formation.

### EXAMPLES

The present invention can be illustrated by the following, non-limiting examples. Unless otherwise specified, percentages given below for solids in solid mixtures, liquids in liquids, and solids in liquids are on a wt/wt, vol/vol and wt/vol basis, respectively.

### Inhibition of IL-2-dependent Neoplastic Cell Proliferation

### Example 1 -- IL-10 inhibits proliferation of human Th0, Th1 and Th2 "like" clones following activation by anti-CD3 monoclonal antibodies crosslinked on mouse L cells transfected with CD32.

### Cells and cell lines.

The T cell lines used were CD2⁺,CD3⁺,CD4⁺, and CD8⁻. Clones HY-06, 827 and 837 have been described previously [Yssel, *et al., Eur. J. Immunol. 16*:1187 (1986) and Haanen, *et al., J. Exp. Med. 174:*583 (1991)]. T cell clone 827 recognizes tetanus toxin (TT) in the context of HLA-DR3 and T cell clone HY-06 recognizes peptide 2-12 from the 65 kD heat shock protein of *Mycobacterium leprae.* The antigen specificity of T cell clone 837 is not known.

T cell clones SP-B21 and SP-A3 were obtained from a patient suffering from severe combined immunodeficiency (SCID) who was successfully reconstituted by fetal thymus and fetal liver transplantation (Roncarolo, *et al., J. Exp. Med.* *168*:2139 (1988)). T cell clones NP 12, NP 14 and NP 44 were established from PBMC of an atopic patient and proliferate specifically in response to the p89-117 peptide of the *Der p* I molecule, a major allergen of the house dust mite (Yssel, *et al., J. Immunol. 148*:738 (1992)). T cell clones CR253, CR378, CR380, AP74, and AP75 were isolated from PBMC from patients suffering from chronic Lyme arthritis and are reactive with *Borrelia burgdorferi* 60 kD heat shock protein homologue (HSP60) [Shanafelt, *et al., J. Immunol. 146*:3985 (1991)].

These T cell clones have been assigned to T helper subsets based on their lymphokine production profiles (Table 1). T cell clones (2x10⁵ cells/ml) were stimulated at two-week intervals by a feeder cell mixture consisting of 10⁶ irradiated (4000 rad) allogeneic peripheral blood leukocytes (PBL) per ml, 10⁵ irradiated (5000 rad) cells per ml of the Epstein-Bar Virus transformed B cell line (EBV-LCL) JY, and 0.1 mg/ml purified PHA (Wellcome Diagnostics, Beckenham, Kent, UK) in 24 well Linbro plates (Flow, Mc Lean, VA), as described previously [Spits, *et al., J. Immunol. 128*:95 (1982)]. Three to four days after each restimulation, the cultures were split and further expanded in medium containing 20 U/ml rIL-2. All cloned T cell lines and EBV-LCL were cultured in Yssel's medium (Yssel, *et al., J. Immunol. Methods* 72:219 (1984)), supplemented with 1% human AB⁺ serum.

Mouse L cells transfected with CD32 (FcγRII) (16.2CG7) were prepared as described in Peltz, *J. Immunol. 141*:1891 (1988), which is incorporated herein by reference. Briefly, an FcγRII cDNA clone, 16.2, isolated from human monocytic cell line U937 mutant was used (see, Stuart *et al., J. Exp. Med. 166*:1668 (1987). Using standard site specific mutagenesis techniques, a mutant lacking the cytoplasmic domain was constructed by introducing mutations which altered the two lysine codons, after the first amino acid (arginine) of the predicted cytoplasmic domain, to stop codons. Transient transfection of mouse L cells was preformed according to standard techniques.

### Reagents.

Recombinant IL-10 was expressed in *E. coli* and purified according to the methods described in de Waal Malefyt *et al., J. Exp. Med. 174*:915 (1991) and WO/91/00349, *supra.*

### Proliferation assays.

The cloned T cells were used 10 to 12 days after stimulation with feeder cells. At this stage the T cell clones were small and in a "resting" state. T cell clones (2x10⁴ cells/well) were incubated with CD32 transfected L cells (2x10⁴ cells/well) in the presence of anti-CD3 mAbs (SPV-T3b) (1 µg/ml) [Spits, *et al., Hybridoma 2*:423 (1983)] in 200 µl flat-bottom plates (Falcon, Becton Dickinson, Lincoln Park, NJ). The L cells were preincubated with the mAbs (1 µg/ml) for 1 hr in the presence or absence of IL-10 (100 U/ml) before the T cells were added and cultured for 72 hours. L cells were treated with mitomycin C (50 mg/ml) (Sigma Chemical Co. St. Louis, MO) at 37°C for 45 min and subsequently washed four times before use. Cells were incubated for 72 h at 37°C and 5% CO₂, pulsed with [³H]TdR for 4 h and harvested as described previously in Yssel, *et al., Eur. J. Immunol. 16*:1187 (1986). The results were expressed as cpm of [³H]TdR incorporation and represent the mean of triplicate cultures.

The effects of IL-10 on the proliferation of the T cell clones did not differ depending on the T cell lymphokine production patterns. T cell clones 837, SP-B21, and 827 produce IL-2, IL-4, IFN-γ, upon activation and therefore they are considered to represent human Th0 clones. HY-06, CR 253, CR 378, CR 380, AP 74, and AP75 produce high levels of IFN-γ and no detectable or low levels of IL-4 and IL-5. These clones are considered to represent human Th1 like clones, whereas NP 12 and NP 44 represent human Th2 like clones, since they produced high levels of IL-4 and IL-5 and no detectable or low levels of IFN-γ following activation by their specific antigen. T cell clone SP-A3 produced IL-2, IL-5, IFN-γ, and GM-CSF, but not IL-4 following activation.

As shown in Table 1, IL-10 inhibited the proliferation of T cell clones 837, SP-B21, SP-A3, 827, HY-06, CR 253, CR 378, CR 380, AP 74, AP 75, NP 12 and NP 44 following activation. Thus, IL-10 was able to inhibit the proliferation of T cell clones belonging to all T helper subsets. Generally, IL-10 inhibited the proliferation of the T cell clones under the present test conditions by 20 - 50 %. This inhibition was dose dependent and specific since it could be reversed by the neutralizing anti-IL-10 mAb 19F1.

**Table 1.**

| Effect of IL-10 on the proliferative responses of human Th0, Th1, and Th2 clones following activation by anti-CD3 mAbs crosslinked on CD32 (FcγRII) transfected L cells. | | | |
|---|---|---|---|
| | [³H]TdR incorporation (cpm x 10⁻³) | | |
| Type T cell clone | T cell clone | control | +IL-10 (100U/ml) |
| T helper 0 | 837 | 69 | 51 |
| | SP-B21 | 88 | 51 |
| | 827 | 5.5 | 3.5 |
| | SP-A3 | 28 | 12 |
| T helper 1 | HY-06 | 11 | 8 |
| | CR 253 | 12.8 | 10 |
| | CR 378 | 14.9 | 4.3 |
| | CR 380 | 19.8 | 8.5 |
| | AP 74 | 22 | 9 |
| | AP 75 | 38 | 33 |
| T helper 2 | NP 12 | 72 | 49 |
| | NP 44 | 15.3 | 7 |

Murine IL-10, which is species specific, had no effect on the proliferation of these human T cell clones. This indicates that IL-10 acts directly on the T cell clones and not via the CD32 transfected mouse L cells used to crosslink the anti-CD3 mAbs. These results show that IL-10 directly inhibited proliferation of human Th0, Th1 and Th2 clones following activation via the TCR/CD3 complex by anti-CD3 mAbs crosslinked on L cells transfected with CD32.

### Example 2 -- IL-10 inhibits proliferation of CD4⁺ T cell clones independently of costimulatory signals provided by ICAM-1, LFA-3, or B7.

Activation of human T cell clones can be modulated by interaction of accessory molecules on T cells with their counterstructures on antigen presenting cells (APC). Interactions between LFA-1 and ICAM1, CD2 and LFA3, CD28 and B7, or CTLA-4 and B7 have been shown to provide costimulatory signals enhancing proliferation and effector functions of T cells. To determine whether IL-10 inhibited T cell proliferation by affecting the costimulatory function of these accessory molecules L cells expressing CD32 and ICAM-1, LFA-3, or B7 were constructed.

### Construction of pCD-SRα-LFA-3 HYG, pCDM8-ICAM-1 HYG and pBJ-B7 HYG.

LFA-3 cDNA was obtained from a Raji cDNA library by PCR amplification using LFA-3 specific primers. The Raji cDNA library was constructed in the pCD-SRa vector as described previously. Matsui *et al., Science 154*:1788 (1991). The following primers were used for cloning of the transmembrane form of LFA-3:

sense: 5'-GGCTGCAGCGACGAGCCATGGTTGCTGGGAGCGACGCG-3'
(nt 1-30) and

antisense: 5'-TTCATCTTCTGGTACCAATCAATTGGAGTTGGTTC-3'
(nt 780-745).

The LFA-3 sense primer was designed with Pst-I site and the LFA-3 antisense primer with Kpn-1 site for convenient subcloning of the amplified products. Amplification was carried out under the following conditions: 1µg of Hind-III digested plasmid DNA of the Raji library was amplified in a 50µl reaction containing 25nmole of each primer, 125mM of each dGTP, dATP, dCTP and dTTP (Pharmacia, Uppsala, Sweden), 50mM KCl, 10 mM Tris-HCl, pH 8.3, 1.5mM MgCl₂ 1mg/ml gelatin, and 5 units *Taq* polymerase (IBI, New Haven, CT).

The mixtures were incubated in a Perkin-Elmer/Cetus DNA Thermal cycler for 20 cycles (denaturation 30s 94°C, annealing 30s 55°C, extension 60s 72°C). An aliquot of 5µl was removed and subjected to a second round of amplification under the same conditions. The reaction was extracted with CHCl₃ and loaded on 1% agarose gels. The amplified product with the expected size of 785bp was cut from the gel after separation, isolated by absorption on silica using a Geneclean kit (Bio 101, La Jolla, CA), cut with Pst-1 and Kpn-1 and subcloned in Bluescript II KS (+) (Stratagene, La Jolla, CA).

A number of clones containing LFA-3 were isolated by standard techniques and 4 clones were sequenced by the dideoxy termination method using a Sequenase DNA sequencing kit (USB, Cleveland, OH). All 4 clones were 100% identical to the published sequence. The Pst-1-Kpn-1 fragment of one of these clones was isolated and subcloned in the pCD-SRa 296 expression vector Matsui et al., *supra.*

pCDM8-ICAM-1 [Blanchard *et al., J. Immunol., 138*:2417 (1987)] was a gift of Dr. Brian Seed (Massachusetts General Hospital, Boston, MA). The aminocyclitol phosphotransferase gene which encodes for hygromycin-B resistance was placed under control of the SV-40 promoter and polyadenylation signals and inserted in the Sfi-I sites of pCD-SRα-LFA-3 and pCDM8-ICAM-1 by standard techniques, yielding pCD-SRα-LFA-3 HYG and pCDM8-ICAM-1 HYG, respectively. pB7-B7-HYG contains human B7 under control of the SRα promotor, Makgoba *et al., Nature 331*:86 (1988), and was a gift of Dr. L. Lanier (DNAX Research Institute).

L cells expressing HLA-DR3 or CD32 were transfected with pCD-SRα-LFA-3-HYG, pCDM8-ICAM-1-HYG or pBJ-B7-HYG by lipofection (BRL, Gaithersburg, MD) according to the manufacturers procedures. Cells were split 48 hr after transfection and medium containing 200µg/ml Hygromycin-B (Lilly, Indianapolis, IN) was added and changed every three days.

After 12 days individual hygromycin-B resistant colonies were visible. These cells were pooled, stained for expression of LFA-3, ICAM-1 or B7 by indirect immunofluorescence and purified by two successive rounds of positive sorting on a FACS-Star Plus. The cotransfected cell lines were maintained in RPMI-1640 supplemented with 200µg/ml hygromycin-B and 10% FCS.

For FACS analysis, cells were incubated in V-bottom microtiter plates (Flow Laboratories, McLean, VA) with purified mAb (10 µg/ml) for 30 min at 4°C. After two washes with PBS containing 0.02mM sodium azide and 1% BSA (Sigma, St. Louis, MO), the cells were incubated with 1/40 dilution of FITC labelled F(ab')2 fragments of goat anti-mouse antibody (TAGO, Inc., Burlingame, CA) for 30 min at 4°C. After three additional washes, the labelled cell samples were analyzed by flow microfluorimetry (FMF) on a FACScan (Becton Dickinson, Sunnyvale, CA). An anti-LFA-3 mAb TS 2/9 was used which has been described in Krensky, *et al., J. Immunol. 132*:2180 (1984). An anti-ICAM-1 mAb LB2 and an anti-B7 mAb L307 were used which have been described in Azuma, *et al., J. Exp. Med. 175*:353 (1992).

The T cell clones were activated by anti-CD3 or anti-CD2 mAbs crosslinked on L cells coexpressing CD32 and ICAM-1, LFA-3 or B7 in the absence or presence of IL-10. It was found that the proliferation of T cell clones SP-B21 and NP 12 was enhanced when clones were stimulated by anti-CD3 mAbs presented by CD32 transfected L cells which coexpressed ICAM-1 and LFA-3. Coexpression of LFA-3 was more efficient in enhancing proliferation of T cell clones as compared to ICAM-1 or B7 and resulted consistently in a 2 to 4 fold increase in proliferation. Only a moderate enhancement in proliferation was observed by coexpression of ICAM-1. B7 generally did not affect the proliferation of T cell clones.

IL-10 inhibited the proliferative responses of T cell clones following activation by anti-CD3 mAbs crosslinked on L cells transfected with CD32 even in the presence of ICAM-1, LFA-3 and B7. However, the inhibition by IL-10 of the proliferation of T cell clones activated by L CD32 cells expressing LFA-3 was less pronounced.

Proliferation of T cell clones could also be induced by a mitogenic combination of anti-CD2 mAbs crosslinked on L CD32 cells, for T cell clones SP-B21 and NP-12. T cell clones (2x10⁴ cells/well) were stimulated by the anti-CD2 mAbs X11-1 (0.5 mg/ml) and D66 (0.5 mg/ml), in 200 µl round-bottom plates (Linbro, Flow Laboratories, Mc. Lean, VA) as described above. The production of these antibodies is described in Brottier, J. *Immunol. 135*:1624 (1985).

T cell proliferation was not affected when ICAM-1 or LFA-3 were coexpressed. However, a moderate enhancement of proliferation was observed when L cells coexpressing CD32 and B7 were used. The anti-CD2 induced proliferation of T cell clones was also inhibited by 30 - 50 % in the presence of IL-10 when L cells expressing CD32 and ICAM-1, LFA-3, or B7 were used. IL-10 did not affect the expression of LFA-1, CD2, or CD28 by the T cell clones. Taken together, these results indicated that the direct inhibitory effects of IL-10 on T cell proliferation were not a result of changes in costimulatory signals provided by ICAM-1, LFA-3, or B7.

### Example 3 -- IL-2, but not IL-4, can reverse the inhibition of T cell proliferation by IL-10.

To determine whether addition of IL-2 or IL-4 could reverse the inhibition of T cell proliferation induced by IL-10, T cell clones were activated by anti-CD3 or anti-CD2 mAbs crosslinked on L cells expressing CD32 and ICAM-1, LFA-3, or B7 in the presence of IL-2 or IL-4. IL-2 was added at 20 U/ml and IL-4 at 200 U/ml. Purified human r-IL-4 (specific activity 2 x10⁷ U/mg) was provided by Schering-Plough Research (Bloomfield, NJ).

It was found that addition of IL-2 at low concentrations could completely reverse the inhibitory effects of IL-10 on the proliferation of T cell clones SP-B21 and NP-12. However, IL-4 added at concentrations of 200 U/ml could not reverse the inhibitory effects of IL-10 on T cell proliferation. Culturing T cell clones for up to 72 hrs in the presence of IL-10 did not result in a downregulation of IL-2 receptor α or β chain expression. In addition, activation of T cell clones with PHA in the presence of IL-10 had no effect on the induction of IL-2 receptor *α* and β chain expression. An anti-IL-2Rα mAb BB10 was used which is described in Herve, *et al., Blood 75*:1017 (1990). An anti-IL-2Rβ mAb Tu 27 was also used which is described in Takeshita, *J. Exp. Med. 169*:1323 (1989). Taken together, these results indicated that the inhibition of T cell proliferation by IL-10 could be reversed by IL-2, but not by IL-4, and likely resulted from an inhibition of IL-2 production.

### Example 4 -- IL-10 inhibits IL-2, but not IL-4, IL-5, IFN-γ and GM-CSF production by human T cell clones.

To determine whether the direct inhibitory effect of IL-10 on the proliferation of T cell clones was due to an inhibition of IL-2 production, T cell clones 837, SP-B21, 827, HY-06, CR 253, NP 12, and NP 44 were activated by anti-CD3 mAbs crosslinked on L cells transfected with CD32 and ICAM-1, LFA-3, or B7 in the absence or presence of IL-10 for 24 hrs, and the production of IL-2, IL-4, IL-5, IFN-γ and GM-CSF was measured by cytokine-specific ELISA. The sensitivity of these ELISA's was: IL-2, 10 pg/ml; IL-4, 50 pg/ml; IL-5, 50 pg/ml; IFN-γ, 300 pg/ml; and GM-CSF, 50 pg/ml.

T cell clones were collected for lymphokine production assays 10 - 12 days after stimulation with feeder cells as described above. Cells were incubated at 37° C in an humidified atmosphere of 5% CO₂ for 24 hrs, culture supernatants were harvested, spun at 250 x g, aliquoted, and stored at -20°C prior to testing.

Activation of T cell clones resulted in the production of IL-2, IL-4, IL-5, GM-CSF, and IFN-γ according to their appropriate Th cell subset cytokine production profiles. Generally the highest levels of cytokine production were measured when T cell clones were activated by L cells expressing CD32 and LFA-3 or B7. In fact, the production of IL-2 was detected predominantly in supernatants of T cell clones activated by anti-CD3 on L CD32-LFA-3 and L CD32-B7 transfectants. IL-10 strongly inhibited the production of IL-2 by the T cell clones. The production of IL-4 was not affected by IL-10, whereas the production of IL-5, GM-CSF, and IFN-γ was only slightly inhibited by IL-10. Thus IL-10 specifically inhibited IL-2 production by activated human T cell clones.

### Example 5 -- IL-10 inhibits IL-2 production by human T cell clones following polyclonal activation.

To further determine whether IL-10 was able to directly act on human T cell clones to inhibit lymphokine production, T cell clones 827, SP-B21 and NP 44 were activated by Tetradecanoylphorbol acetate (TPA) (Calbiochem), anti-CD3 mAbs, and TPA, PHA and TPA, or a mitogenic combination of anti-CD2 mAbs in the absence of accessory cells. T cell clones were stimulated at concentrations of 1 x 10⁶ cells/ml, PHA (100 ng/ml), anti-CD3 mAb SPV-T3b (1 µg/ml), and TPA (1 ng/ml).

As shown in Table 2, polyclonal activation of T cell clones 827, SP-B21, and NP 44 resulted in high levels- of lymphokine production according to their lymphokine production profiles. IL-10 strongly inhibited the production of IL-2 by these clones at all modes of activation tested, whereas the production of IL-4, IL-5, IFN-γ, and GM-CSF was not affected. IL-10 was able to inhibit IL-2 production by these T cell clones following activation by anti-CD3 + TPA and following Ca⁺⁺ ionophore + TPA, indicating that the mechanism of inhibition does not involve T cell receptor/CD3 signal transduction pathways and that this mechanism acts downstream of protein kinase C activation.

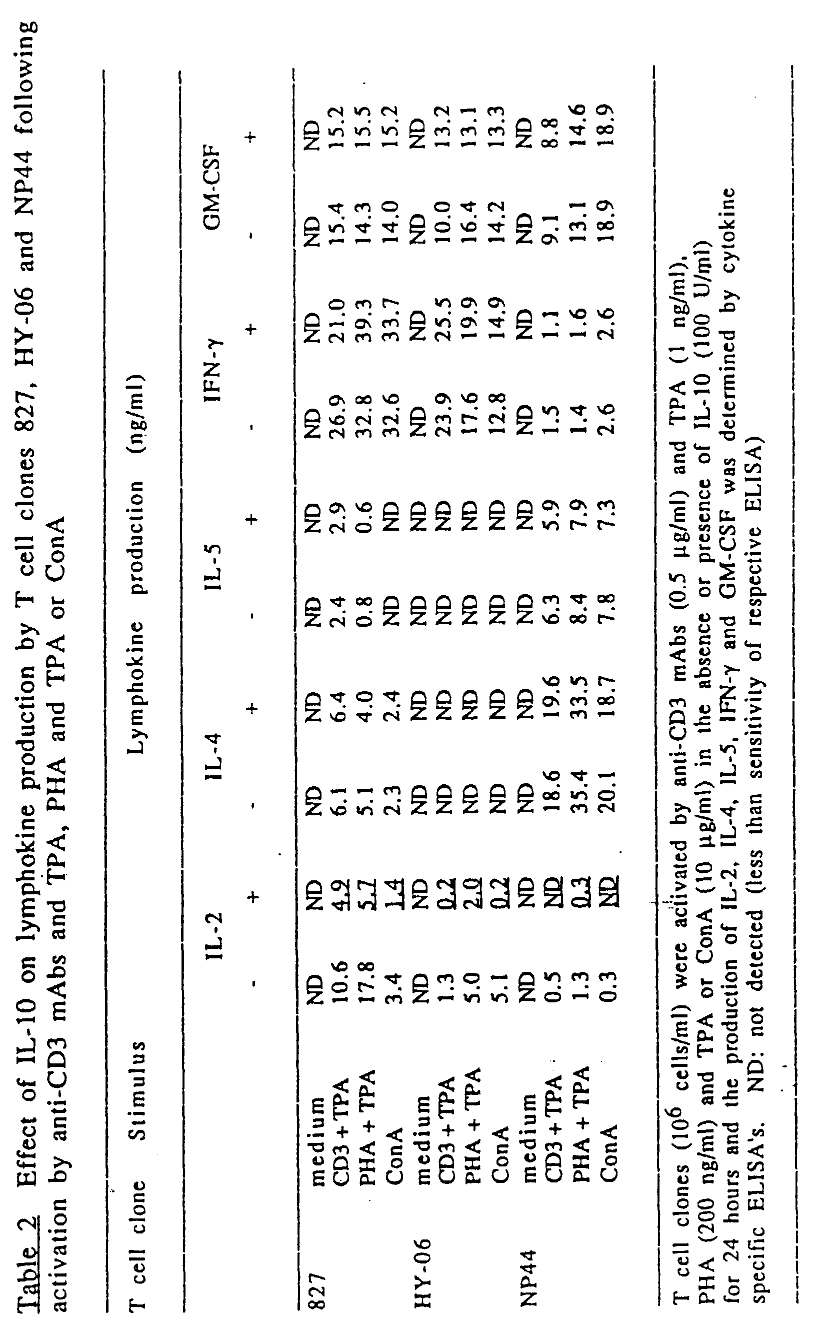

### Example 6 -- IL-10 inhibits IL-2 production by human T cell clones at the mRNA level.

To determine at what level IL-10 inhibited the production of IL-2, the expression IL-2, IL-4, IL-5, and IFN-γ mRNA was analyzed by Northern blots in T cell clones NP-12, HY-06, 827, SP-A3, 837, and SP-B21 following activation by anti-CD3 mAbs and TPA in the absence or presence of IL-10. Total RNA was isolated by the method of Chirgwin, *et al., Biochem.* 18:5294 (1979). Northern analyses were performed as described in de Waal Malefyt, J. *Immunol. 142*:3634 (1989).

The following probes were used for northern analysis: 285 bp Pst-I - Xba-I fragment (nt 1 - 285) of pCD-hIL-2, which is described in Yokota, *et al. In Lymphokines* 13 (1987); 318bp Nhe-I - EcoRI fragment of pCD-hIL-4 (nt 106 - 424 ), which is described in Yokota, *et al., Proc. Natl. Acad. Sci.* USA 83:5894 (1986); 1100 bp Pst-I - Hinc-II fragment of pCD-hIFN-γ (nt 5 - 1106) Yokota, *supra;* and 1200 bp Pst-I fragment of pAL described in de Waal Malefyt, *et al., J. Immunol. 145*:2297 (1990).

IL-10 strongly inhibited the levels of IL-2 mRNA expressed in these clones but did not affect the expression of IL-4 and IL-5 by Th0- and Th2-like clones, or the expression of IFN-y by Th0- and Th1-like clones. These results indicated that IL-10 specifically inhibited the production of IL-2 by human T cell clones at the mRNA level.

### Example 7 -- IL-10 inhibits IL-2 production by T cells isolated from peripheral blood cells.

Whether IL-10 could inhibit proliferation and IL-2 production by resting T cells was determined. T cells were isolated from peripheral blood and activated by anti-CD3 or anti-CD2 mAbs crosslinked on CD32 transfected L cells alone or CD32 L cells which coexpressed ICAM-1, LFA-3 or B7. Total PBMNC were isolated from buffy coats of healthy donors by centrifugation over Ficoll-Hypaque (Sigma Diagnostics, St. Louis, MO) density gradients as described by Boyum, A., *Scan. J. Clin. Lab. Invest. 21* (Suppl 97):77 (1968).

T cells were purified from PBMNC following plastic adherence, passage over nylon wool columns and negative selection by magnetic depletion. Briefly, 100 x 10⁶ PBMNC were cultured for 30 min at 37°C in a 100 mm tissue culture dish (Becton Dickinson, Lincoln Park, NJ) in Yssel's medium supplemented with 1 % human AB serum. Non-adherent cells were removed and passed over a nylon wool (Robbins Scientific, Sunnyvale, CA) column [Julius, *et al. Eur. J. Immunol.* 3:645 (1973)]. Cells were subsequently incubated with saturating concentrations of anti- CD14 (Leu M3), CD16 (Leu 11a), CD19 (Leu 12), and CD56 (Leu 19) mAbs for 30 min at 4°C, washed and incubated with sheep-anti-mouse IgG coated magnetic beads (Dynabeads M450, Dynal A.S., Oslo, Norway) at a bead to cell ratio of 40:1.

The mixture was incubated with gentle shaking for 30 min at 4°C and rosetted cells were removed with the magnetic particle concentrator according to manufacturer's recommendations. The T cell populations were 97-99% CD2⁺, CD3⁺. Anti-CD14 (Leu-M3), CD16 (Leu 11a), CD19 (Leu 12), and CD56 (Leu 19) mAbs and control mAbs of the appropriate isotypes were purchased from Becton Dickinson, Mountain View, CA.

PBMNC T cells (2x10⁵ cells/well) were stimulated by anti-CD3 or anti-CD2 mAbs crosslinked on LFcγRII cell transfectants as described above. Culture supernatants were harvested after 24 hrs of incubation at 37°C.

It was found that resting T cells were completely dependent on the presence of the B7 antigen on the CD32 L cells for activation. Both anti-CD3 or anti-CD2 mAbs crosslinked on CD32 L cells were ineffective in inducing T cell proliferation and lymphokine production. In addition, these cells remained ineffective following cotransfection of ICAM-1 or LFA-3 into these cells. However, anti-CD2 or anti-CD3 mAbs crosslinked to CD32 L cells that had been cotransfected with B7 induced strong T cell proliferation and high levels of IL-2, IFN-γ and GM-CSF production. IL-10 did not significantly inhibit the proliferation of the resting T cells under these conditions. In contrast, IL-10 did inhibit the production of IL-2, but not the production of IFN-γ and GM-CSF by these cells.

The levels of IL-2 produced in the presence of IL-10, however, were still sufficient to induce maximal proliferation at the time point test (Day 3). Collectively these results indicated that engagement of CD28 or CTLA-4 on the T cells by interaction with B7 on the accessory cell was absolutely required for induction of proliferation and lymphokine production by resting T cells, and that IL-10 was still able to inhibit IL-2 production under these conditions.

### Example 8 -- IL-10 inhibits antigen specific T cell proliferation when mouse L cells transfected with human HLA molecules are used as APC.

The effects of IL-10 on the antigen specific proliferation of the Tetanus Toxoid (TT) specific T cell clone 827 and M. Leprae 65 kD hsp pt [2-12] specific T cell clone HY-06 were examined when mouse L cells transfected with HLA-DR3 or HLA-DR3 and ICAM-1, LFA3 or B7, as described above, were used as antigen-presenting cells. The FACS profiles of the expression of HLA-DR, ICAM-1, LFA3 and B7 by the L cell transfectants showed that IL-10 had no effect on the constitutive expression of class II MHC on these cells.

Tetanus Toxin was provided by Dr. B. Bizzini (Institute Pasteur, Paris, France) and used at a final concentration of 1 µg/ml. Hsp pt [2-12] was made by a solid phase peptide synthesis methodology and checked using analytical reverse phase HPLC and amino acid analysis. The peptides were used at a final concentration of 0.5 µg/ml.

The stimulation of T cell clones 827 and HY-06, proliferation assays and cytokine measurements were performed as described above. It was found that IL-10 inhibited the antigen specific proliferation of HY-06 and 827 with L cells transfected with HLA-DR3 as antigen presenting cells (APC), in a dose dependent fashion. IL-10 added at 10 U/ml had inhibitory activity, and up to 30-50% inhibition of proliferation was obtained at 100 U/ml and 500 U/ml.

These results were confirmed when L cells transfected with HLA-DR and ICAM-1, LFA3 or B7 were used as APC. The proliferative responses of T cell clones 827 and HY-06 increased when L cells cotransfected with LFA3 were used, and the inhibitory effect of IL-10 was less pronounced. The inhibition of the proliferative responses of 827 and HY-06 by IL-10 was completely reversed by the neutralizing anti-IL-10 mAb 19F1, indicating the specificity of the inhibition. These results indicated that IL-10 inhibited antigen specific proliferative responses of 827 and HY-06 when HLA-DR transfected mouse L cells were used as APC.

The inhibition of antigen specific proliferation by IL-10 was found to be at the T cell level. To determine this, T cell clones 827 and HY-06 were activated by TT and pt [2-12] in the presence of human or murine IL-10, to investigate whether IL-10 was acting on the antigen presenting L cells or directly on the T cell clones. As discussed above, human IL-10 is active on human and mouse cells, but murine IL-10 is only active on mouse cells.

It was found that murine IL-10 was not able to inhibit the antigen specific proliferation of 827 and HY-06 when L cells transfected with HLA-DR alone or cotransfected with ICAM-1, LFA3 or B7 were used as APC. These results indicated that IL-10 had a direct effect on the proliferation of human T cell clones.

T cell clones 827 and HY-06 were activated by TT or pt [2-12] with L cells expressing HLA-DR alone or in combination with ICAM-1, LFA3 or B7 in the presence or absence of IL-10 and IL-2, to examine whether the addition of exogenous IL-2 could reverse the inhibitory effects of IL-10. Low concentrations of IL-2 could reverse the inhibitory effects of IL-10 on antigen specific proliferation of T cell clones 827 and HY-06.

T cell clone HY-06 was activated by pt [2-12] with L cells expressing HLA-DR3 alone or in combination with ICAM-1, LFA3 or B7 in the presence or absence of IL-10, and the production of IL-2 and IFN-γ was determined in the culture supernatants harvested at 24 hours. IL-10 inhibited the production of IL-2, but not of IFN-γ by T cell clone HY-06 following antigen specific activation. Taken together these results indicated that IL-10 inhibited antigen-specific T cell proliferation by a specific inhibition in the production of IL-2.

### Inflammatory Bowel Disease

A murine model of IBD has been developed. This model contributes to elucidation of the etiology or mechanism of IBD. Additionally, it provides an experimental forum for various therapeutic agents. To develop the model, the IL-10 gene is either removed or rendered ineffective or non-expressible in the experimental animal. The animal is referred to as having the IL-10 gene "knocked out" or as being a "knockout" (KO) animal. The KO can be accomplished by any of several means known in the art.

For example, a transgenic animal whose IL-10 gene has been inactivated can be produced. *See* Goodnow, C., "Transgenic Animals," 1502-1504, *Encyclopedia of Immunology,* Roitt (ed.), Academic Press, San Diego (1992). Briefly, purified DNA is microinjected into the pronuclei of fertilized oocytes. Afterwards, the oocytes are surgically implanted into the oviducts of foster mothers. Alternatively, standard genetic breeding techniques may be used to isolate a homozygous mutant animal strain.

Additionally, there are methods which inactivate specific genes in the germ line of mice. In this case, a mutant transgene is introduced in place of the wild-type gene through homologous recombination. Embryonal stem (ES) cell lines "can be isolated and culture *in vitro* from mouse blastocysts, and later reimplanted into another blastocyst so as to partly colonize both the somatic and germ-line tissues of the resulting mouse." Goodnow at 1503.

DNA is introduced in the ES cells in culture and the blastocysts are surgically implanted in a foster mother. Heterozygous mutant progeny result which are mated to each other so that, ultimately, offspring having two mutant alleles, e.g., a homozygous mutant, are produced. These offspring would have no functional alleles of the gene of interest and allow for "genetic dissection" to remove specific genes. Analysis of the resulting offspring can provide insight into the role of the targeted gene in the KO mice in causing resultant phenotypes.

For additional methods of creating an experimental animal having deletion of a particular gene, *see* McMahon, *et al.,* "The Midbrain-Hindbrain Phenotype of *Wnt-1*^{*-*}*|Wnt-I*^{*-*} Mice Results from Stepwise Depletion of *engrailed*-Expressing Cells by 9.5 Days Postcoitum," *Cell 69*:581 (1992) and Travis, "Scoring a Technical Knockout in Mice," *Science 256*:1392 (1992). Additional applicable technologies include the art of mutagenesis and antisense technologies. *See* Goodnow, C. (1992, *supra).*

### Example 9 -- Murine Model for Inflammatory Bowel Disease.

Mice which were devoid of the IL-10 gene were raised. See Kuhn *et al. Science 254*:707 (1991); Capecchi, *Science* *244*:1288 (1989); Robertson (ed.) *Teratocarcinomas and embryonic stem cells: A Practical Approach,* I.R.L. Press, Oxford (1987); and Zijestra, *et al. Nature 336*:348 (1989), for descriptions of the general technology and for the procedures to make mice devoid of a selected gene. Mice which lack the IL-10 gene are referred to below as IL-10T mice, or knock out (KO) mice.

First Group. Four mice were sacrificed for bone marrow assays. A portion of their marrow was analyzed to determine their ability to produce myeloid and erythroid progenitors (see Table 3) and to assess production of stem cells (see Table 4). A remaining portion of the marrow was used for long-term bone marrow cultures to assess the capability of micro-environmental cells to support normal hematopoietic generation. The thymus and spleen contained normal numbers of T-cells and normal numbers of B-cells. The short-term progenitor and stem-cell assays are given in tabular form below. The cells from the controls and the knock-out mice were separately pooled.

**Table 3.**

| Number of Colony-Forming Cells/Femur | | | | | | |
|---|---|---|---|---|---|---|
| | Total cells/femur | GM | BFU-e | CFU-e | Meg | Meg/Mix |
| Control | 9.5x10⁶ | 18,430 | 475 | 42,500 | 2,470 | 285 |
| IL-10T | 9.4x10⁶ | 21,902 | 940 | 59,740 | 2,271 | 658 |
| GM = granulocytes and macrophages; BFU = blood forming units; CFU = colony forming units; -e = erythroid; Meg = megakaryocyte colonies; Meg/Mix = megakaryocytes mixed with other lineages.Colony-stimulating factors used in assay: IL-3 + IL-6 + erythropoietin (Epo) | | | | | | |

**Table 4.**

| Numbers of Day-14 CFU-S | |
|---|---|
| Control group | 1,678 per femur |
| IL-10T group | 1,517 per femur |
| Stem cell production is the number of CFU-S in a femur of the mouse. CFUS measures the formation of hemopoietic nodules in the spleen of lethally irradiated mice following transplantation. See Till *et al., Radiation Research 14*:213 (1961). | |

The IL-10T mice did not show any obvious defect in the production of myeloid, erythroid and stem cells. Concurrently, the long-term cultures had formed a layer of supportive stroma in all cultures including those from IL-1OT mice. These cultures were set up in two stages: First, cells were put into culture to form the complex stromal layers needed to support continuous hematopoiesis. Then the cultures were reseeded with additional bone marrow cells from equivalent types of donors to provide the actual precursors that associate with the stromal cells. This second stage was undertaken when the second and third groups of mice were available.

Second and third groups. The bone marrow cells from these animals were needed to complete the long-term bone marrow culture experiments. In addition to using their bone marrow for this purpose, other procedures were performed (see Tables 5-9). The IL-10T animals were anemic but seemed to have normal levels of bone marrow precursors. Therefore, their anemia was evaluated and their organs were observed histologically.

**Table 5.**

| Peripheral Blood Counts | | | |
|---|---|---|---|
| Mice | WBC/ml x 10⁶ | RBC/ml x 10⁹ | Platelets/ml x 10⁸ |
| Control #1 | 7.2 | 7.3 | 12.6 |
| #2 | 11.2 | 5.6 | 10.7 |
| #3 | 10.0 | 6.7 | 8.5 |
| #4 | 8.2 | 5.6 | 9.8 |
| #5 | 7.7 | 5.6 | 8.0 |
| | | | |
| IL-10T #1 | 4.0 | 2.4 | 20.6 |
| #2 | 4.8 | 7.1 | 8.0 |
| #3 | 7.5 | 3.9 | 27.3 |
| #4 | 6.5 | 5.5 | 23.9 |
| WBC = white blood cells, RBC = red blood cells | | | |

**Table 6.**

| Peripheral Blood Differentials | | | | | |
|---|---|---|---|---|---|
| Mice | % Lymphs | % Neut | % Mono | % Eos | % Retics |
| Control #1 | 87 | 12 | 1 | 0 | 2.4 |
| #2 | 72 | 26 | 2 | 0 | 1.2 |
| #3 | 80 | 18 | 1 | 1 | 0.6 |
| | | | | | |
| IL-10T #1 | 36 | 64 | 0 | 1 | 3.8 |
| #2 | 40 | 57 | 0 | 3 | 5.3 |
| #3 | 23 | 77 | 0 | 0 | 17.1 |
| Lymphs = lymphocytes, Neut = neutrophils, Mono = monocytes, Eos = eosinophils, Retics = reticulocytes | | | | | |

RBC Morphology. All control animals had normal appearing red blood cells (RBC's). The IL-10T mice showed variable morphologies. IL-10T #1 had microcytic cells; #2 and #3 had microcytic cells plus some macrocytic, polychromic cells and occasional nucleated red blood cells (NRBC's). Red blood smears were stained with new methylene blue and the cells showing ribosomal staining were enumerated. Positive staining occurs in very young red blood cells (reticulocytes) and implies premature release from hemopoietic organs. This is referred to as the reticulocyte count.

Organ Histology of IL-10T Mice. Inspection of sectioned heart, lung, kidney and thymus appeared normal. The liver appeared normal except for occasional foci of mononuclear and neutrophilic granulocytes. The spleens were grossly abnormal. Follicles were present but the red pulp area was filled with hematopoietic cells, mostly NRBC's and blast forms (immature cells). There were few or no mature RBC's and there was no iron storage with the macrophages.

**Table 7.**

| Spleen Cell Differentials | | | | | | | | |
|---|---|---|---|---|---|---|---|---|
| | (% of total cells) | | | | | | | |
| Mice | Cells/spleen | Lymph | Mono | Neu | Eos | Plasma Cell | Blast | Nuc RBC |
| Control #1 | 1.5x10⁸ | 88 | 2 | 1 | <1 | <1 | 1 | 6 |
| #2 | 1.5x10⁸ | 89 | 1 | <1 | <1 | <1 | 2 | 7 |
| #3 | 1.1x10⁸ | 79 | 3 | 4 | 1 | 2 | 2 | 8 |
| | | | | | | | | |
| IL-10T #1 | 2.1x10⁸ | 64 | 2 | 7 | 1 | 1 | 2 | 22 |
| #2 | 2.5x10⁸ | 54 | 2 | 13 | 3 | 1 | 6 | 21 |
| #3 | 1.6x10⁸ | 43 | 2 | 10 | 1 | 1 | 10 | 32 |
| Lymph = lymphocytes, Mono = monocytes, Neu = neutrophils, Eos = eosinophils, Retics = reticulocytes, Blast = blastocyte, Nuc RBC = nucleated red blood cell | | | | | | | | |

**Table 8.**

| Bone Marrow Differentials | | | | | | | | |
|---|---|---|---|---|---|---|---|---|
| | (% of Total) | | | | | | | |
| Mice | Neu | Neu MB | Eos | Eos MB | Nuc RBC | Plasma Cell | Lym | Blast Undif |
| Control #1 | 46 | 12 | 10 | 4 | 20 | <1 | 4 | 4 |
| #2 | 39 | 9 | 5 | 3 | 37 | <1 | 5 | 2 |
| #3 | 35 | 10 | 12 | 4 | 29 | <1 | 6 | 2 |
| | | | | | | | | |
| IL-10T #1 | 46 | 12 | 7 | 2 | 28 | <1 | 2 | 2 |
| #2 | 56 | 19 | 4 | 1 | 18 | <1 | 1 | 1 |
| #3 | 53 | 14 | 2 | 1 | 27 | 1 | 1 | 1 |
| Neu MB = neutrophilic myeloblast; Eos MB = eosinophilic myeloblast; Nuc RBC = nucleated RBC; Blast Undif = primitive undifferentiated blastocyte; Lym = lymphocytes | | | | | | | | |

The following data are the results of colony-forming assays. The assays were done with spleen cells as well as bone marrow cells. IL-3 + c-kit ligand instead of IL-3 + IL-6 was used to stimulate the colony-forming cells. The combination of IL-3 + c-kit ligand is more effective in stimulating erythroid precursors as evidenced by comparing the numbers of BFU-e obtained in the first assay (above) with the numbers of BFU-e obtained in the following assays.

**Table 9.**

| Colony-Forming Assays | | | | | | | |
|---|---|---|---|---|---|---|---|
| Mice | GM | GEM | GEMM | BFU-e | CFU-e | MEG | MEG/MIX |
| Spleen (colonies/spleen) | | | | | | | |
| C1 | 18,535 | 618 | 309 | 9,268 | 42,322 | 618 | 1,236 |
| C2 | 7,736 | 893 | 595 | 2,678 | 44,926 | 1,488 | 893 |
| C3 | 8,670 | 650 | 650 | 7,152 | 71,741 | 433 | 433 |
| | | | | | | | |
| K1 | 55,020 | 8,400 | 3,360 | 51,660 | 393,120 | 2,100 | 6,300 |
| K2 | 111,060 | 25,746 | 10,601 | 96,925 | 498,762 | 14,135 | 12,116 |
| K3 | 68,274 | 20,096 | 9,890 | 103,050 | 1,012,633 | 14,357 | 9,252 |

| Bone Marrow (colonies/femur) | | | | | | | |
|---|---|---|---|---|---|---|---|
| C1 | 37,300 | 3,632 | 838 | 7,963 | 35,065 | 978 | 419 |
| C2 | 49,159 | 2,552 | 510 | 5,954 | 81,478 | 680 | 1,021 |
| C3 | 30,343 | 1,916 | 639 | 6,069 | 24,754 | 958 | 958 |
| | | | | | | | |
| K1 | 63,440 | 1,823 | 1,641 | 8,750 | 58,336 | 1,276 | 1,276 |
| K2 | 37,340 | 6,570 | 438 | 8,870 | 47,085 | 986 | 1,424 |
| K3 | 35,432 | 4,006 | 1,753 | 14,273 | 52,208 | 1,628 | 4,132 |
| C1, C2, and C3 refer to the same control mice also designated Control #1, #2, and #3. K1, K2, and K3 refer to the same mice also designated IL-10T #1, #2, and #3. GM = granulocyte, macrophage GEM = granulocyte, erythoid, macrophage GEMM = granulocyte, erythoid, macrophage, megakaryocyte | | | | | | | |

**Table 10.**

| Occult Blood Tests | | |
|---|---|---|
| Mice | day 1 | day 2 |
| Control #1 | neg | neg |
| #2 | neg | neg |
| #3 | no sample | neg |
| | | |
| IL-10T #1 | neg | neg |
| #2 | pos | pos |
| #3 | pos | pos |

Examination of Feces. Studies of the feces of the IL-10T mice did not reveal evidence of eggs suggesting parasitic infection. There was no evidence of mature parasites residing in the upper or lower intestine.

The animals varied between slightly and markedly anemic. The anemia cannot be explained by inability to generate erythrocytes. The IL-10T mice showed signs of trying to compensate for the anemia. First, they produced larger than normal numbers of RBC precursors in their spleens. In mice, the spleen normally would try to compensate for RBC deficiencies. The murine spleens were two times larger than in normal animals and they were overactive in trying to generate RBC's.

Some of the mice were compensating well. They had near normal numbers of RBC's in their blood and, they had not released many immature cells into circulation because their reticulocyte numbers were relatively normal. However, other mice fell behind and they pushed cells into the circulation prematurely which is why their reticulocyte counts were so high (i.e., 17%). They also had a few circulating nucleated RBC's.

These mice also had higher than normal platelet counts as well as elevated numbers of megakaryocytes in their spleens. This was a sign that they may have been bleeding. Loss of blood would account for the chronic anemia with the depletion of iron stores (this iron deficiency was evident in both the spleen and bone marrow compartments). Upon autopsy, it was clear that there was no internal bleeding. However, most of the IL-10T mice showed blood in their feces and also showed prolapsed rectums. The mice were examined closely and found to be negative for pinworms, tapeworms, or other parasitic infections. There was a direct correlation between positive blood in the feces and the severity of their anemia.

In addition to the anemia and bleeding problem, the IL-10T mice had more neutrophilic granulocytes in their blood than lymphocytes. This is the opposite of what is normal for mice. See the peripheral blood differential (Table 6). This observation is also consistent with a huge increase in myeloid precursors found in the spleen colony-forming assays Table 9). Thus, the mice suffered from the syndrome "anemia of chronic inflammation."

At autopsy, tissue sections prepared from IL-10T mice and litter-mate controls were examined. All specimens from the controls appeared normal. Referring to the IL-10T mice, the thymus and spinal tissue appeared normal. The spleen showed mild to marked increase in erythroblasts and myeloblasts plus megakaryocytes. RBC pools were small or essentially nonexistent. Prussian blue staining showed depletion of iron stores and there was evidence of increased extramedullary hematopoiesis.

Depletion of iron stores is consistent with blood loss and results in abnormal erythropoiesis. On gross examination, the spleens were one and one-half to three times normal size. The mesenteric lymph nodes were very large compared to normal and were almost as large as a normal spleen. B and T-cell follicles were similarly enlarged, and this picture is consistent with an inflammatory response.

The small intestine showed well-formed mucosa and submucosa. In some sections, the numbers of polymorphonucleated white cells (PMN) and mononuclear cells were slightly increased. The large intestine and rectum revealed marked inflammation of the rectum extending proximally including the lower portion of the colon. A marked amount of edema and infiltrating cells were evident. PMN's were the predominant cell type, but eosinophils, lymphocytes, plasma cells, and epithelioid cells (cells which resemble epithelium) were present. Inflammation involved the mucosa, crypts and submucosa. Occasionally, infiltrates were found in the muscularis layer, especially in the internal sphincter region. In many areas, the epithelium was lost or absent. Multiple lesions showed fibrous material with aggregated RBC's and PMN's. Some blood vessels were congested with RBC's. Clumps of epithelioid cells suggested early formation of granuloma, but no well-formed granulomas were evident. There was no evidence of parasites, eggs or intracellular bacteria, which can cause chronic inflammation. The multiple lesions and hemorrhage were consistent with the positive result of the stool occult blood test. The entire picture was consistent with IBD with secondary anemia and wasting.

### Example 10 -- IL-10 KO Mice Respond to Treatment With Exogenous IL-10.

Four mice which were devoid of the IL-10 gene (IL-10 KO) and three normal control mice were examined. The control mice had a normal physical appearance. All three of the control mice had negative occult blood stool samples and had normal-appearing rectums. Some of the IL-10 KO mice were noticeably smaller than the controls and they showed signs of poor health. They had ruffled hair and poor vigor compared to the normal controls. One of the four IL-10 KO mice was hunched and walked with an abnormal gait. All four of the IL-10 KO mice had swollen rectums and either erythematous rectal tissue or definite rectal prolapse.

Two of the experimental KO mice had definitely positive occult blood stool samples on two separate occasions, whereas the third mouse was negative on one occasion and equi,vocal on a second occasion. Rectal smears were performed on all four KO mice and three had purulent exudates with large numbers of neutrophilic granulocytes. Erythrocytes were also present on individual smears of the animal that was bleeding from the rectum at the time of sampling. Because IL-10 KO mice showed wasting with time, the weight of the IL-10 KO mice and the normal controls were recorded as part of the examination.

Three of the IL-10 mice were injected daily for two weeks with 35 micrograms of IL-10. These three mice had consistently shown purulent exudates (only 2 out of the 3 were also showing signs of bleeding and 1 out of the 3 was hunched up and walked abnormally). One IL-10 KO mouse (negative for bleeding and a purulent exudate) and the normal controls were injected daily for two weeks with tromethamine (TRIS) buffer. The general health, physical appearance, rectal exudate, occult blood and weight were monitored.

After the second week of IL-10 infusion, all three IL-10 KO mice showed increased vigor, smoothed hair, and normal posture and walk. All three showed reduced rectal swelling, although signs of inflammation were still present. All three had negative occult blood results and there were no signs of erythrocytes in their rectal smears. The amount of exudate was reduced; however, neutrophilic granulocytes were still observed in their rectal smears. The smear of one animal now showed more epithelial and lymphocyte cells than neutrophilic granulocytes. One animal maintained its weight at approximately 16 grams. The other two increased their weight from 16 to 17.8 grams and 11.4 to 14 grams.

The IL-10 KO mouse that received TRIS buffer for two weeks still had a negative occult blood stool sample. It showed abundant exudate which contained large numbers of neutrophilic granulocytes. The rectum was still swollen and susceptible to prolapse. The animal was hunched up and walked abnormally. It also showed obvious signs of wasting, and its weight had dropped from 16.6 to 14 grams. The normal controls which had also been injected with TRIS buffer retained a normal health appearance including their rectums. Their occult blood stool samples were still negative and there were no rectal exudates detected. Their weight increased from 17.1 to 19 grams and 17.4 to 18.7 grams. Even several weeks later, the animals continued to gain weight.

### Example 11 -- Amelioration of Signs or Symptoms of Attributable to Inflammatory Bowel Disease.

A human patient having IBD can be treated with IL-10 according to the invention as follows. Such a patient typically has symptoms which may include diarrhea, abdominal pain, fever, melena, hematochezia, and weight loss and signs which may include abdominal mass, glossitis, aphthous ulcer, anal fissure, perianal fistula, anemia, malabsorption, and iron deficiency. The patient is initially treated with five µg of IL-10 per kilogram body weight per day. Because the patient weighs 70 kg, the initial starting dose is 350 µg per day of IL-10. This dose is administered as an intravenous bolus. The dose is increased by about 50 to 150 µg per day depending on the patient's tolerance and response. An optimum dose of about 700 µg per day (10 µg per kilogram per day) is achieved after several days.

Prior to the first treatment, and daily thereafter until several days after the final treatment, the patient is monitored clinically and with laboratory parameters. The laboratory parameters include blood counts with particular attention to the total white blood cell count and its differential. Of special interest is whether the numbers of white cells remain normal or without significant change from the patient's pre-treatment level. With respect to the white cell differential, particular attention is given to whether immature forms are appearing in the peripheral blood and whether the normal ratio of different types of white cells is constant or changing. Special attention is paid to the ratio of lymphocytes and monocytes in the peripheral blood.

Additional parameters which can be monitored include the erythrocyte sedimentation rate (ESR), chemical profiles, blood levels of IL-6, TNF and IFN-γ. Finally, regular, such as daily, evaluation of the patient's stool for blood and purulent exudate can indicate the patient's response to therapy. Treatment results in improvement of one or more symptoms or signs of the intestinal inflammation.

### Inhibition of Delayed-type Hypersensitivity Reactions

### Example 12 -- Decreased Effects in Mice.

BALB/c mice were pretreated with anti-CD4 monoclonal antibody (MoAb) and four weeks later were infected with *Leishmania major.* BALB/c is a commercially available inbred strain of mice obtained from Simonsen Laboratories (Gilroy, California). The mice used were 8 to 12 weeks of age. *L. major* (WHO strain designation WHOM/-/173) were cultured as promastigotes in M199 (available from GIBCO, Grand Island, NY) containing 30% fetal calf serum (FCS) (available from J.R. Scientific, Woodland, CA), 2mM L-glutamine, and 100U/ml each of penicillin and streptomycin. Promastigotes were harvested from stationary phase cultures and washed in phosphate buffered saline (PBS). Mice were infected with 1.5 x 10⁷ viable promastigotes injected subcutaneously into the left hind footpad. *L. major* antigen was prepared by four cycles of freezing and thawing of the parasites followed by centrifugation. The antigen preparation was added to culture wells at an equivalent of 2 x 10⁶ organisms/ml.

The following monoclonal antibodies (McAbs) were used for *in vitro* assays: a neutralizing anti mouse IL-4 MoAb (ATCC No. HB188), a neutralizing anti mouse IL-10 McAb [prepared by the method for anti-IL-4 disclosed by Chatelain, *et al., J. Immunol. 148*:1182 (1992)], and anti mouse CD4 (ATCC No. TIB207). All antibody preparations were purified from tissue culture supernatants by ion exchange chromotography and gel filtration and contained less than 3 endotoxin units (EU)/mg protein, as measured by the Limulus agglutination assay.

For cell purifications, the following purified McAbs were used: biotinylated RM-4-5 and RM-4-4, anti mouse CD4 and AMS-32.1, anti mouse Mac-1 (ATCC No. TIB128), RA3-6B2, and anti mouse B220 [Coffman, *Immunological Reviews 69*:5 (1982)]. Recombinant mouse IL-4 and IL-10 were expressed in *E. coli* and affinity purified as described previously.

Popliteal lymph nodes (LN) draining the site of *L. major* infection were removed 4 to 6 weeks post infection from BALB/c or BALB/c anti-CD4 pre-treated mice and teased into single cell suspensions in PBS containing 0.25% bovine serum albumin (BSA). For purification of CD4+ T cells, all manipulations were carried out at 4°C. Approximately 4 x 10⁸ LN cells were labelled with 2 ml of a PBS/BSA solution containing 12 µg/ml each of anti-B220, anti-Mac-1, anti-CD8 and anti-class II I-A^{d} for 30 min. The McAb labelled cell suspension, after washing in PBS/BSA, was then incubated on a rotating wheel for 30 min, with sheep anti-rat coated Dynabeads (Dynabeads are available from Robbins Scientific, Mountain View, Calif.) at a ratio of 3 beads per cell. Negative cells were obtained by magnetic separation.

CD4+ T cells were further enriched from the resulting cell suspension, which was about 85% CD4+, by positive selection using a magnetic activated cell sorter (MACS, Miltenyi, Dusseldorf, Germany). Briefly, cells were labelled with 12 µg/ml biotinylated anti-CD4 in PBS/BSA, about 50 ml/10⁸ cells, for 15 minutes. The cells were washed in PBS and incubated with Streptavidin Microbeads (from Becton Dickinson, Sunnyvale, Calif.) at about 50 µl/10⁸ cells. After 15 minutes, Streptavidin-PE (from Becton Dickinson) was added at a final concentration of 1/50. After a further 5 minutes incubation, the cell suspension was washed and passed through a magnetic column (MACS, Miltenyi, Dusseldorf, Germany), according to the manufacturer's instructions. Cells which were retained on the magnetized column were eluted by removing the column from the magnet and washing extensively with PBS/BSA. The resulting cell population was greater than 96% CD4+.

T cell-depleted splenocytes were prepared from the spleens of normal donors from which anti-CD4 and anti-CD8 staining cells were removed by negative selection using sheep anti-rat Dynabeads exactly as described for LN cells above. Preparations were routinely 99% CD4 and CD8 negative by FACS analysis.

Unseparated lymph node cells or purified CD4+ T cells were set up in duplicate cultures in cRPMI containing 5% FCS at the indicated doses in 250 µl volumes in 96 well round bottomed plates, together with, in the case of purified CD4+ T cells, 5 x 10⁵ cell depleted normal splenocytes in the presence or absence of L. *major* antigen. cRPMI refers to a complete culture media containing RPMI-1640 (available from J.R.H. Biosciences in Lenexa, Kansas), 10% FCS, 2 mM L-glutamine, 0.05 mM 2-ME and 100 U/ml each of penicillin -and streptomycin.

Antigen presentation cells (APC) were pulsed with *L. major* antigen (equivalent to 2 x 10⁶ organisms/ml) or medium alone for 4 hours. The T cell depleted splenocytes were then exposed to 1000 rad γ-radiation prior to culture. In some cases, recombinant IL-10 (rIL-10) (50 µg/ml), recombinant IL-4 (rIL-4) (100 ng/ml), and/or neutralizing McAbs to these cytokines were added to the culture wells. Supernatants were harvested after 72 hours for detection of cytokine synthesis.

Cytokine levels in supernatants were detected by two-site sandwich ELISA as described above for IFN-γ (see Chatelain *et al.* 1992, *supra),* IL-4 (Chatelain *et al.* 1992, *supra),* IL-10 and IL-3. Samples were quantified by comparison with standard curves of purified recombinant or natural cytokine.

The pretreatment with anti-CD4 MoAb initially depleted the animal's available T cells, but ultimately enhanced the animal's resistance to the parasitic challenge by *L. major.* Although this phenomenon was counter-intuitive and its mechanism was unclear, it was widely observed. DTH strongly correlated with the animal's resistance to parasitic infection.

Four weeks after infection, the animals were challenged with Leishmania freeze-thawed antigen in the contralateral footpad. Over the next 48 hour period, footpad swelling was monitored with a metric caliper. Each animal received five doses of cytokines intraperitoneally. One group received five doses of IL-4, another group received five doses of IL-10 and a third group received five doses of a combination of IL-4 and IL-10. Each of the five doses was given every 12 hours with the first dose at 12 hours before the antigenic challenge.

**Table 11.**

| Combination of IL-4 and IL-10 Inhibits the DTH to *L. major* Antigens | |
|---|---|
| Treatment | Footpad Size (mm x 10⁻²) |
| 20 µg IL-4 | 84 ± 16 |
| 20 µg IL-10 | 68 ± 7 |
| 20 µg IL-4 + 20 µg IL-10 | 33 ± 11 |
| 4 µg IL-4 + 4 µg IL-10 | 54 ± 25 |
| PBS control | 91 ± 20 |

Table 11 shows mouse footpad size 24 hours after injection of Leishmania antigen. The response of control animals injected with PBS alone is compared with the response of animals which received IL-4, IL-10 or a combination of both at the indicated doses. Cytokines were administered intraperitoneally (IP) every 12 hours for 48 hours, starting 12 hours prior to antigenic challenge. There were five animals in each group.

The data demonstrate that mice treated with IL-4 alone fared about the same as mice treated with PBS. Mice treated with IL-10 alone had less swelling than the controls.

Mice treated with a combination of IL-4 and IL-10 responded with the least swelling, particularly the group treated with 20 µg of each cytokine.

**Table 12.**

| Combination of IL-4 and IL-10 Inhibits the DTH to *L. major* Antigens | |
|---|---|
| Treatment | Footpad Size (mm x 10⁻²) |
| 50 µg IL-4 | 73 ± 11 |
| 50 µg IL-10 | 65 ± 12 |
| 50 µg IL-4 + 50 µg IL-10 | 43 ± 13 |
| 20 µg IL-4 + 20 µg IL-10 + ICA | 42 ± 9 |
| 20 µg IL-4 + 20 µg IL-10 + αIL-10 + αIL-4 | 93 ± 21 |
| PBS Control | 119 ± 27 |

Table 12 shows data from a second independent experiment. The experimental format was the same as described above, except that footpad data were taken at 48 hours after the injection of Leishmania antigen.

There were five animals in each group. The inhibitory effect of IL-4 and IL-10 was blocked by administration of 2 mg of a monoclonal antibody against IL-4 and 5 mg of a monoclonal antibody against IL-10. The antibodies against IL-4 and IL-10, used as positive controls, were given IP 12 hours before the first administration of cytokines. 5 mg of an isotype control monoclonal antibody (ICA), added as a negative control, had no effect on the inhibition. ICA was prepared as described by Chatelain, et al, 1992 (previously cited).

The antibodies against IL-4 and IL-10 were added to show that competition for IL-4 and IL-10 reduced the effectiveness of the cytokines. Also, these antibodies provided a further control for the recombinantly-produced cytokines.

The data indicate that the combination of IL-4 and IL-10 is more effective than either cytokine alone in reducing an animal's DTH reaction. IL-10 alone is more effective than IL-4 alone, but less effective than the combination. Administration of the combination of cytokines with monoclonal antibodies raised against each cytokine resulted in negation of the effectiveness of the cytokine treatment.

**Table 13.**

| IFN-γ Production from LN Cells Draining the Site of DTH Induction | |
|---|---|
| Treatment | IFN-γ (ng/ml) |
| 20 µg IL-4 | 147 ± 34 |
| 20 µg IL-10 | 109 ± 49 |
| 20 µg IL-4 and 20 µg IL-10 | 53 ± 33 |
| PBS Control | 242 ± 54 |

The data of Table 13 were produced by administering cytokines every 12 hours up to 24 hours post-antigen challenge, starting 12 hours prior to antigen challenge. Cells from lymph nodes (LN) draining the site of Leishmania antigen injection were removed five days after antigen challenge from animals that received cytokines and antigen. The column labelled "Treatment" in Table 13 refers to the treatment received by the animal from which the LN cells were taken. The lymph nodes were stimulated *in vitro* with *L. major* freeze thawed antigen. The supernatant was tested for IFN-γ content 72 hours later. Data shown are from three individual animals (n = 3 ± standard error).

The data show that animals treated with cytokine yielded less IFN-γ than the controls treated with PBS. Moreover, animals treated with the combination of IL-4 and IL-10 revealed considerably less IFN-γ than any of the control of the experimental animals which received a single cytokine.

**Table 14.**

| IL-4 and IL-10 Inhibition of IFN-γ Production by a Th1 Population | |
|---|---|
| Addition | IFN-γ (ng/ml) |
| none | 100 ± 6 |
| 5 ng/ml IL-10 | 53 ± 4 |
| 100 ng/ml IL-4 | 44 ± 2 |
| 5 ng/ml IL-10 and 100 ng/ml IL-4 | 20 ± 1 |

To produce the data of Table 14, CD4+ T cells (3.5 x 10⁵/well) were isolated from anti-CD4 pretreated mice infected with *L. major* four weeks earlier. Cells were stimulated *in vitro* with *L. major* freeze thawed antigen together with T cell depleted splenocytes (5 x 10⁵/well) as a source of APC. IFN-γ levels were determined after 72 hours by ELISA. The column labelled "Addition" refers to substances added to the media in which the Th1 cells were cultivated.

The data of Table 14 show that both IL-4 and IL-10 administered alone significantly diminished levels of IFN-y produced by Th1 cells, as compared with controls treated with PBS. Moreover, addition of the combination IL-4 and IL-10 to the media resulted in about 50% less IFN-γ than did the addition of either cytokine alone.

The foregoing data show that IL-10 consistently diminished the DTH response, swelling, and IFN-γ production from lymph nodes generally and from Th1 cells specifically, as compared to controls. Furthermore, the combination of IL-4 and IL-10 was superior to either IL-4 or IL-10 alone at the same dose range. The combination therapy at 20 µg for each cytokine showed results superior to single therapy at more than twice the dose (50 µg) for each of IL-4 and IL-10. Thus, 20 µg each of IL-4 and IL-10 given as combination therapy showed benefits superior to 50 µg of either IL-4 or IL-10, given as a single therapeutic agent.

### Example 13 -- Treatment of a Patient Having Diabetes Mellitus Type I.

A patient having diabetes mellitus type I, or insulin-dependent diabetes mellitus, as diagnosed using either the criteria developed by the National Diabetes Data Group or the World Health Organization is selected for treatment. The patient is initially treated with 20 µg of each of IL-4 and IL-10 per kilogram body weight per day. Because the patient weighs 70 kg, the initial starting dose is 1400 µg per day of each cytokine. This dose is administered as an intravenous bolus injection. The dose is increased by about 250 to 1000 µg per day depending on the patient's tolerance and response. An optimum dose of about 7000 µg per day (100 µg per kilogram per day) is achieved after several days.

Prior to the first treatment, and daily thereafter until several days after the final treatment, the patient is monitored clinically and with laboratory parameters. The laboratory parameters include blood glucose and blood counts with particular attention to the total white blood cell count and its differential. Blood glucose monitoring is well known in the treatment of diabetics. Of additional interest is whether the amount of white cells remains normal or without significant change from the patient's pre-treatment level. With respect to the white cell differential, particular attention is given to whether immature forms are appearing in the peripheral blood and whether the normal ratio of different types of white cells is constant or changing. Special attention is paid to the ratio of lymphocytes and monocytes in the peripheral blood.

Many modifications and variations of this invention can be made without departing from its spirit and scope, as will become apparent to those skilled in the art. The specific embodiments described herein are offered by way of example only, and the invention is to be limited only by the terms of the appended claims.

## Claims

1. The use of IL-10 in the manufacture of a pharmaceutical composition for inhibiting delayed-type hypersensitivity reaction.

2. The use of Claim 1 wherein the composition is to be used in combination with IL-4.

3. The use of IL-4 in the manufacture of a pharmaceutical composition for inhibiting delayed-type hypersensitivity reaction, wherein the composition is to be used in combination with IL-10.

4. The use according to Claim 2 or Claim 3 wherein the IL-10 and IL-4 are administered simultaneously of sequentially.

5. A pharmaceutical composition capable of inhibiting delayed-type hypersensitivity reaction, comprising IL-4 and IL-10 and a pharmaceutically acceptable carrier.

6. A method of producing a pharmaceutical composition capable of inhibiting delayed-type hypersensitivity reaction, which comprises admixing IL-4, IL-10 and a pharmaceutically acceptable carrier.

7. The use, pharmaceutical composition or method of any preceding claim wherein the IL-10 is human IL-10.

8. The use, pharmaceutical composition or method of any preceding claim wherein the IL-4 is human IL-4.
